# EUROPEAN PATENT SPECIFICATION

(11) **EP 4 301 880 B1**
(45) Date of publication and mention of the grant of the patent: **15.07.2026**
(21) Application number: 22763658.6
(22) Date of filing: 07.03.2022
(51) Int. Cl.: C12Q 1/6858

(54) **METHOD FOR DETECTION OF SARS-COV-2 MUTATIONS**
VERFAHREN ZUM NACHWEIS VON SARS-COV-2-MUTATIONEN
MÉTHODE POUR LA DÉTECTION DE MUTATIONS DU SARS-COV-2

(30) Priority: 05.03.2021 KR 20210029741
(43) Date of publication of application: 10.01.2024
(73) Proprietor: Seegene, Inc., Seoul 05548 (KR)
(72) Inventor: KIM, Yun Jee, Seoul 05232 (KR); BAE, Ju Hee, Seoul 05820 (KR); SONG, Young Ho, Gyeonggi-do 14786 (KR); JIN, So Dam, Gyeonggi-do 11917 (KR)
(74) Representative: Kilger, Ute
(86) International application number: PCT/KR2022/003219
(87) International publication number: WO 2022/186677

(56) References cited:
- WO-A1-2021/222827
- WO-A1-2022/159874
- WO-A1-2022/171584
- CN-A- 111 304 372
- CN-A- 111 440 896
- CN-A- 111 445 955
- VOGELS CHANTAL B ET AL: "Multiplexed RT-qPCR to screen for SARS-COV-2 B.1.1.7 variants: Preliminary results - SARS-CoV-2 coronavirus / nCoV-2019 Diagnostics and Vaccines - Virological", 1 January 2021 (2021-01-01), XP093272533, Retrieved from the Internet <URL:https://virological.org/t/multiplexed-rt-qpcr-to-screen-for-sars-cov-2-b-1-1-7-variants-preliminary-results/588>
- KORUKLUOGLU GULAY, KOLUKIRIK MUSTAFA, BAYRAKDAR FATMA, OZGUMUS GOZDE GIRGIN, ALTAS AYSE BASAK, COSGUN YASEMIN, KETRE KOLUKIRIK CAN: "40 minutes RT-qPCR Assay for Screening Spike N501Y and HV69-70del Mutations", BIORXIV, 26 January 2021 (2021-01-26), XP055847139, Retrieved from the Internet <URL:https://www.biorxiv.org/content/10.1101/2021.01.26.428302v1.full.pdf> DOI: 10.1101/2021.01.26.428302
- CADENA JUAN FERNÁNDEZ, MUÑOZ MINDY, LEÓN GABRIEL MOREY, ARMAS-GONZÁLEZ RUBÉN, MÁRQUEZ DARLYN AMAYA, VITERI KATHERYN SACHERI, CONSO: "Detection of the new SARS-CoV-2 variant B.1.526 with the Spike E484K mutation in South America", RESEARCH SQUARE, 17 February 2021 (2021-02-17), XP055962943, DOI: 10.21203/rs.3.rs-248965/v1

## Description

### [Technical Field]

This application claims priority to and the benefit of Korean Patent Application No. 10-2021-0029741 filed in the Korean Intellectual Property Office on 05 March 2021.

The present disclosure relates to a method for detection of SARS-CoV-2 mutations.

### [Background Art]

Molecular diagnostics is currently a rapidly growing field in the in-vitro diagnostic market for early diagnosis of diseases. Among the techniques, methods using nucleic acids are useful in diagnosing causative genetic factors for viral and bacterial infection on the basis of the high specificity and sensitivity thereof.

Diagnostic methods using nucleic acids are, for the most part, inclusive of amplification of target nucleic acids (for example, viral or bacterial nucleic acids). Representative of the nucleic acid amplification methods is the polymerase chain reaction (PCR), which includes repeated cycles of denaturation of double-stranded DNA, annealing of oligonucleotide primers to a DNA template, and extension of the primers by DNA polymerase (Mullis et al., U. S. Patent Numbers 4,683,195, 4,683,202, and 4,800,159; and Saiki et al., (1985) Science 230, 1350-1354).

Various methods have been for amplifying nucleic acids as exemplified by ligase chain reaction (LCR), strand displacement amplification (SDA), nucleic acid sequence-based amplification (NASBA), transcription mediated amplification (TMA), and rolling-circle amplification (RCA). Vogels Chantal B et al., URL:https://virological.org/t/multiplexed-rt-qpcr- to-screen-for-sars-cov-2-b-1-1-7-variants-preliminary-results/588 describes an open-source multiplexed PCR that recreates the TaqPath SGTF signature.

In recent years, real-time PCR diagnostic kits for detecting SARS-CoV-2 have attracted great keen interest with the worldwide spread of severe acute respiratory syndrome coronavirus 2 (SARS-CoV-2).

SARS-CoV-2 is an RNA virus belonging to the Coronaviridae family, with various mutations reported therefor. The mutations include a silent mutation in which, in spite of alteration in the nucleotide sequence, no amino acid changes happen so that the viral biological characteristics remain intact, and a missense mutation in which a nucleotide change brings about a codon encoding for a different amino acid which leads to a protein misfolding and a structural change, with the consequent alteration of biological characteristics in the virus. Recently, SARS-CoV-2 variants with missense mutations have been reported to have much higher powerful infectivity than preexisting SARS-CoV-2 viruses. In such situations, active research has been conducted particularly into spike proteins.

Spike proteins play a pivotal role in viral entry into human cells. According to reports, mutations in the spike gene encoding for spike proteins make it easier for viruses to invade human cells and thus infect humans. The vaccines currently developed for SARS-CoV-2 mainly target spike proteins. It is reported that mutations in such spike proteins are likely to incapacitate the efficacy of the vaccines.

Indeed, SARS-CoV-2 variants that have recently spread worldwide at highly rapid rates include mutations in spike genes encoding for spike proteins and have reduced vaccine effectiveness.

Accordingly, there is a need for a method that can prevent the spread of viral transmission early by quickly detecting SARS-CoV-2 having a mutation in a spike protein which may affect the infectivity and vaccine effectiveness.

Numerous documents and patent documents are referred to herein over the entire specification and indicated as referred to.

### [Disclosure]

### [Technical Problem]

The present inventors have conducted thorough and intensive research into the development of a method for detecting SARS-CoV-2 mutations which cause an increase in viral infectivity or a decrease in vaccine effect.

As a result, the present inventors found that the SARS-CoV-2 mutations, which are able to affect the viral infectivity and vaccine, can be detected by detecting the five mutations (i) HV69/70 del, (ii) Y144 del, (iii) E484K, (iv) N501Y, and (v) P681H, which are located in the spike (S) gene of SARS-Cov-2.

Therefore, the present disclosure provides a method for detecting SARS-CoV-2 mutations in a sample comprising a nucleic acid molecule therein.

The present disclosure also provides a composition for detecting SARS-CoV-2 mutations.

Other purposes and advantages of the present disclosure will be clarified by the following detailed description, together with the accompanying claims.

### [Technical Solution]

According to an aspect thereof, the present disclosure provides a method for detecting SARS-CoV-2 mutations in a sample comprising a nucleic acid molecule, the method comprising the steps of:
(a) incubating the sample with a composition for detecting SARS-CoV-2 mutations; and
(b) detecting the SARS-CoV-2 mutations,

wherein the composition comprises a plurality of PTOs (Probing and Tagging Oligonucleotide) hybridizing respectively with nucleotide mutation sequences of interest located in the S gene of SARS-CoV-2, ^
wherein the PTO comprises (i) a 3'-targeting portion comprising a hybridizing nucleotide sequence complementary to the target nucleic acid sequence, and (ii) a 5'-tagging portion comprising a nucleotide sequence non-complementary to the target nucleic acid sequence;
wherein the nucleotide mutation sequences of interest each encodes for any one of the following amino acid mutations:
   (i) HV69/70 del,
   (ii) Y144 del,
   (iii) E484K,
   (iv) N501Y, and
   (v) P681H
wherein (1) said PTO capable of hybridizing with the nucleotide mutation sequence of interest encoding for the amino acid mutation HV 69/70 del includes a nucleotide mutation-discriminating site at the middle region of its 3'-targeting portion, and
(2) said PTO capable of hybridizing with the nucleotide mutation sequence of interest encoding for the amino acid mutation Y144 del includes a nucleotide mutation-discriminating site at the middle region of its 3'-targeting portion;
wherein (1) said PTO capable of hybridizing with the nucleotide mutation sequence of interest encoding for the amino acid mutation E484K includes a nucleotide mutation-discriminating site in a 5'-end region of its 3'-targeting portion,
(2) said PTO capable of hybridizing with the nucleotide mutation sequence of interest encoding for the amino acid mutation N501Y includes a nucleotide mutation-discriminating site in a 5'-end region of its 3'-targeting portion, and
(3) said PTO capable of hybridizing with the nucleotide mutation sequence of interest encoding for the amino acid mutation P681H includes a nucleotide mutation-discriminating site in a 5'-end region of its 3'-targeting portion,
wherein the nucleotide mutation-discriminating site comprises a complementary sequence to the nucleotide variation on the target nucleic acid,
wherein the composition further comprises (i) a pair of primers capable of hybridizing with a nucleic acid sequence comprising the nucleotide mutation sequences of interest encoding respectively for the amino acid mutations HV69/70 del and Y144 del, (ii) a pair of primers capable of hybridizing with a nucleic acid sequence comprising the nucleotide mutation sequences of interest encoding respectively for the amino acid mutations E484K and N501Y, and (iii) a pair of primers capable of hybridizing with a nucleic acid sequence comprising the nucleotide mutation sequence of interest encoding for the amino acid mutation P681H,
wherein the composition further comprises an oligonucleotide capable of hybridizing with a conserved nucleic acid sequence among SARS-CoV-2 wild type and variants and an oligonucleotide capable of hybridizing with a nucleic acid sequence of an internal control.

In an embodiment of the present disclosure, the sample may be a swab, saliva, or a combination thereof.

In an embodiment of the present disclosure, the swab may be a nasopharyngeal swab, a nasal swab, an oropharyngeal swab, a saliva swab, or a combination thereof.

In an embodiment of the present disclosure, the sample may be is a crude extract sample which has not been subjected to any purification of a nucleic acid sequence.

In an embodiment of the present disclosure, the incubation may include a reverse transcription and a nucleic acid amplification reaction.

In an embodiment of the present disclosure, each of the PTOs may hybridize with the nucleotide mutation sequence of interest in a hybridization condition and does not hybridize with a nucleotide sequence without the nucleotide mutation sequence of interest.

Each of the PTOs includes a mutation-discriminating site located in a middle region thereof, as defined in the claims, and as further defined herein below.

In an embodiment of the present disclosure, each of the PTOs may be capable of hybridizing with both the nucleotide mutation sequence of interest and a nucleotide sequence without the nucleotide mutation sequence of interest in a hybridization condition, with different hybridization patterns therebetween at the 5'-end region of the PTOe.

In an embodiment of the present disclosure, step (b) may be performed in a post-amplification detection manner or in a real-time detection manner.

In an embodiment of the present disclosure, the conserved nucleic acid sequence may be located in a nucleic acid sequence selected from the group consisting of E gene, N gene, RdRP gene, and S gene.

The composition further includes an oligonucleotide capable of hybridizing with a nucleic acid sequence of an internal control.

In an embodiment of the present disclosure, the internal control may be RNase P.

Provided according to another aspect of the present disclosure is a composition for detecting SARS-CoV-2 mutations, the composition comprising a plurality of PTOs (Probing and Tagging Oligonucleotide) hybridizing respectively with nucleotide mutation sequences of interest located in the S gene of SARS-CoV-2,
wherein the PTO comprises (i) a 3'-targeting portion comprising a hybridizing nucleotide sequence complementary to the target nucleic acid sequence, and (ii) a 5'-tagging portion comprising a nucleotide sequence non-complementary to the target nucleic acid sequence;
wherein the nucleotide mutation sequences of interest each encodes for any one of the following amino acid mutations:
   (i) HV69/70 del,
   (ii) Y144 del,
   (iii) E484K,
   (iv) N501Y, and
   (v) P681H
wherein (1) said PTO capable of hybridizing with the nucleotide mutation sequence of interest encoding for the amino acid mutation HV 69/70 del includes a nucleotide mutation-discriminating site at the middle region of its 3'-targeting portion, and
(2) said PTO capable of hybridizing with the nucleotide mutation sequence of interest encoding for the amino acid mutation Y144 del includes a nucleotide mutation-discriminating site at the middle region of its 3'-targeting portion;
wherein (1) said PTO capable of hybridizing with the nucleotide mutation sequence of interest encoding for the amino acid mutation E484K includes a nucleotide mutation-discriminating site in a 5'-end region of its 3'-targeting portion,
(2) said PTO capable of hybridizing with the nucleotide mutation sequence of interest encoding for the amino acid mutation N501Y includes a nucleotide mutation-discriminating site in a 5'-end region of its 3'-targeting portion, and
(3) said PTO capable of hybridizing with the nucleotide mutation sequence of interest encoding for the amino acid mutation P681H includes a nucleotide mutation-discriminating site in a 5'-end region of its 3'-targeting portion,
wherein the nucleotide mutation-discriminating site comprises a complementary sequence to the nucleotide variation on the target nucleic acid,
wherein the composition further comprises (i) a pair of primers capable of hybridizing with a nucleic acid sequence comprising the nucleotide mutation sequences of interest encoding respectively for the amino acid mutations HV69/70 del and Y144 del, (ii) a pair of primers capable of hybridizing with a nucleic acid sequence comprising the nucleotide mutation sequences of interest encoding respectively for the amino acid mutations E484K and N501Y, and (iii) a pair of primers capable of hybridizing with a nucleic acid sequence comprising the nucleotide mutation sequence of interest encoding for the amino acid mutation P681H,
wherein the composition further comprises an oligonucleotide capable of hybridizing with a conserved nucleic acid sequence among SARS-CoV-2 wild type and variants and an oligonucleotide capable of hybridizing with a nucleic acid sequence of an internal control.

### [Advantageous Effects]

Features and advantages of the present disclosure are as follows:
(a) Features of the present disclosure rely on the detection of five specific mutations, among many mutations located in the S gene of SARS-CoV-2, which can elevate viral infectivity and have an influence on vaccine effects, in order to quickly screen SARS-CoV-2 that has changed in viral characteristics (e.g., infectivity, mortality, vaccine effect, therapeutic effect, etc.).
(b) The present disclosure aims to rapidly detect SARS-CoV-2 variants that has changed in viral characteristics (e.g., infectivity, mortality, vaccine effect, therapeutic effect, etc.), thus early preventing and promptly coping with the spread of infection of SARS-CoV-2 variants.

### [Best Mode]

The present disclosure provides a method for detecting SARS-CoV-2 mutations in a sample comprising a nucleic acid molecule, the method comprising the steps of:
(a) incubating the sample with a composition for detecting SARS-CoV-2 mutations; and
(b) detecting the SARS-CoV-2 mutations,
   wherein the composition comprises a plurality of PTOs (Probing and Tagging Oligonucleotide) hybridizing respectively with nucleotide mutation sequences of interest located in the S gene of SARS-CoV-2,
   wherein the PTO comprises (i) a 3'-targeting portion comprising a hybridizing nucleotide sequence complementary to the target nucleic acid sequence, and (ii) a 5'-tagging portion comprising a nucleotide sequence non-complementary to the target nucleic acid sequence;
   wherein the nucleotide mutation sequences of interest each encodes for any one of the following amino acid mutations:
      (i) HV69/70 del,
      (ii) Y144 del,
      (iii) E484K,
      (iv) N501Y, and
      (v) P681H
   wherein (1) said PTO capable of hybridizing with the nucleotide mutation sequence of interest encoding for the amino acid mutation HV 69/70 del includes a nucleotide mutation-discriminating site at the middle region of its 3'-targeting portion, and
   (2) said PTO capable of hybridizing with the nucleotide mutation sequence of interest encoding for the amino acid mutation Y144 del includes a nucleotide mutation-discriminating site at the middle region of its 3'-targeting portion;
   wherein (1) said PTO capable of hybridizing with the nucleotide mutation sequence of interest encoding for the amino acid mutation E484K includes a nucleotide mutation-discriminating site in a 5'-end region of its 3'-targeting portion,
   (2) said PTO capable of hybridizing with the nucleotide mutation sequence of interest encoding for the amino acid mutation N501Y includes a nucleotide mutation-discriminating site in a 5'-end region of its 3'-targeting portion, and
   (3) said PTO capable of hybridizing with the nucleotide mutation sequence of interest encoding for the amino acid mutation P681H includes a nucleotide mutation-discriminating site in a 5'-end region of its 3'-targeting portion,
   wherein the nucleotide mutation-discriminating site comprises a complementary sequence to the nucleotide variation on the target nucleic acid,
   wherein the composition further comprises
      (i) a pair of primers capable of hybridizing with a nucleic acid sequence comprising the nucleotide mutation sequences of interest encoding respectively for the amino acid mutations HV69/70 del and Y144 del,
      (ii) a pair of primers capable of hybridizing with a nucleic acid sequence comprising the nucleotide mutation sequences of interest encoding respectively for the amino acid mutations E484K and N501Y, and
      (iii) a pair of primers capable of hybridizing with a nucleic acid sequence comprising the nucleotide mutation sequence of interest encoding for the amino acid mutation P681H,
   wherein the composition further comprises
   an oligonucleotide capable of hybridizing with a conserved nucleic acid sequence among SARS-CoV-2 wild type and variants and
   an oligonucleotide capable of hybridizing with a nucleic acid sequence of an internal control.

Below, a detailed description will be given of the present disclosure according to each step:

### Step (a): Incubation of sample and composition for detecting SARS-CoV-2 mutation

**In the present disclosure, first, a sample comprising a nucleic acid** molecule is incubated with a composition for detecting SARS-CoV-2 mutations.

As used herein, the term "sample comprising a nucleic acid molecule" refers to a sample considered to comprise a target nucleic acid molecule therein.

As used herein, the term "nucleic acid", "nucleotide sequence" or "nucleic acid molecule" refers to a single- or double-stranded deoxyribonucleotide or ribonucleotide polymer in which the nucleotides include derivatives of naturally occurring nucleotides, non-naturally occurring nucleotides, or modified nucleotides, which are capable of functioning in the same manner as naturally occurring nucleotides.

The term "target nucleic acid", "target nucleic acid sequence", or "target sequence", as used herein, refers to a nucleic acid sequence to be detected, which is annealed to or hybridized with a probe or primer in a hybridization, annealing or amplification condition.

The term "primer", as used herein, refers to an oligonucleotide which is capable of acting as a point of initiation of synthesis when placed under such a condition that induces the synthesis of a primer extension product complementary to a target nucleic acid (template), that is, in the presence of nucleotides and an agent for polymerization, such as DNA polymerase, and at suitable temperatures and pH. The primer must be long enough to prime the synthesis of extension products in the presence of the agent for polymerization. The exact length of the primers may vary depending on many factors including, for example, temperatures, field of application, and primer sources.

In an embodiment of the present disclosure, the oligonucleotide may have a length of 5 to 1,000 bp, 5 to 900 bp, 5 to 800 bp, 5 to 700 bp, 5 to 600 bp, 5 to 500 bp, 5 to 400 bp, 5 to 300 bp, 5 to 200 bp, 5 to 150 bp, 5 to 100 bp, 5 to 90 bp, 5 to 80 bp, 5 to 70 bp, 5 to 60 bp, 5 to 50 bp, 5 to 40 bp, 5 to 30 bp, 5 to 20 bp, 5 to 10 bp, 10 to 1,000 bp, 10 to 900 bp, 10 to 800 bp, 10 to 700 bp, 10 to 600 bp, 10 to 500 bp, 10 to 400 bp, 10 to 300 bp, 10 to 200 bp, 10 to 150 bp, 10 to 100 bp, 10 to 90 bp, 10 to 80 bp, 10 to 70 bp, 10 to 60 bp, 10 to 50 bp, 10 to 40 bp, 10 to 30 bp, 10 to 20 bp, 15 to 1,000 bp, 15 to 900 bp, 15 to 800 bp, 15 to 700 bp, 15 to 600 bp, 15 to 500 bp, 15 to 400 bp, 15 to 300 bp, 15 to 200 bp, 15 to 150 bp, 15 to 100 bp, 15 to 90 bp, 15 to 80 bp, 15 to 70 bp, 15 to 60 bp, 15 to 50 bp, 15 to 40 bp, or 15 to 30 bp, but with no limitations thereto.

As used herein, the term "probe", in particular in the context of a PTO means a single-stranded nucleic acid molecule including a portion or portions that are substantially complementary to a target nucleic acid sequence. According to an embodiment of the present disclosure, the 3'-end of the probe may be "blocked" to prohibit its extension. The blocking may be achieved in accordance with conventional methods. For instance, a chemical moiety such as biotin, a label, a phosphate group, an alkyl group, a non-nucleotide linker, phosphorothioate, or alkane-diol may be linked to the 3'-hydroxyl group of the last nucleotide to block the extension therefrom. Alternatively, the blocking may be carried out by removing the 3'-hydroxyl group of the last nucleotide or by using a nucleotide lacking 3'-hydroxyl group, such as dideoxynucleotide.

The primer or the probe may be a single-stranded oligonucleotide. The primer or the probe may include deoxyribonucleotide, ribonucleotide or a combination thereof. The primers or probes used in the present disclosure may comprise naturally occurring dNMP (i.e., dAMP, dGM, dCMP and dTMP), modified nucleotides, or non-natural nucleotides.

The sequence of the primer or probe needs not be perfectly complementary to that of a template. So long as it allows the primer or probe to hybridize with a template to exert its own function, any complementarity is sufficient therebetween.

The term "annealing" or "priming", as used herein, refers to the apposition of an oligonucleotide or nucleic acid to a template nucleic acid, whereby the polymerase can polymerize nucleotides into a nucleic acid molecule which is complementary to the template nucleic acid or a portion thereof.

As used herein, the term, "hybridization" refers to the formation of a double-stranded nucleic acid from two single-stranded polynucleotides through non-covalent bonds between complementary nucleotide sequences under a predetermined hybridization condition.

The oligonucleotide (e.g., primer or probe) of the present disclosure includes a hybridizing nucleotide sequence to a target nucleic acid sequence.

As used herein, the term "a hybridizing nucleotide sequence to a target nucleic acid sequence" means "a nucleotide sequence hybridizing to a target nucleic acid sequence".

The hybridizing nucleotide sequence of an oligonucleotide (e.g., primer or probe) includes a sequence that can hybridize with a target nucleic acid sequence in a given hybridization condition (i.e., a sequence that hybridizes specifically with a nucleotide mutation sequence of interest).

According to an embodiment of the present disclosure, the oligonucleotide (e.g. primer or probe) includes a hybridizing nucleotide sequence that can hybridize with a target nucleic acid sequence in a stringent condition.

In an embodiment of the present disclosure, the stringent condition includes a temperature condition in which a temperature is set forth to be selected within a certain range around a Tm value of a target sequence to be hybridized by the oligonucleotide, for example, a temperature selected from among Tm value ±1°C, ±2°C, ±3°C, ±4°C, ±5°C, ±6°C, and ±7°C.

In an embodiment of the present disclosure, the stringent condition may include the following conditions:
(1) 5XSSC, 5XDenhardt's solution, 0.5% SDS, 50% formamide 32°C, (2) 5XSSC, 5XDenhardt's solution, 0.5% SDS, 50% formamide, 42°C, (3) 5XSSC, 1% SDS, 50 mM Tris-HCl (pH 7.5), 50% formamide 42°C, (4) 5XSSC, 5XDenhardt's solution, 0.5% SDS, 50% formamide 50°C, (5) 0.2XSSC, 0.1% SDS 60°C, (6) 0.2XSSC, 0.1% SDS 62°C, (7) 0.2XSSC, 0.1% SDS 65°C, or (8) 0.1XSSC, 0.1% SDS 65°C, but is not limited thereto.

According to an embodiment of the present disclosure, the hybridizing nucleotide sequence of the oligonucleotide (e.g., primer or probe) may be determined depending on a hybridization condition employed, a nucleic acid sequence to be hybridized by the oligonucleotide, etc.

According to an embodiment, the stringent condition is a temperature at which the entire sequence of the oligonucleotide hybridizes with a target nucleic acid sequence, but not with non-target nucleic acid sequences.

According to an embodiment, the stringent condition is a temperature at which a portion (e.g., 5'- or 3'-terminal region) of the oligonucleotide sequence hybridizes with a target nucleic acid sequence, but not with non-target nucleic acid sequences.

With respect to relationship between two different oligonucleotides, the expression "include(s) (or comprise(s)) a hybridizing nucleotide sequence", as used herein, means that entirety or portion of one oligonucleotide has a complementary nucleotide sequence necessary for hybridization with entirety or portion of the other oligonucleotide.

According to an embodiment of the present disclosure, an oligonucleotide (e.g. primer or probe) may include a hybridizing nucleotide sequence complementary to a target nucleic acid sequence.

As used herein, the term "complementary" mean that primers or probes are complementary enough to hybridize selectively to a target nucleic acid sequence in a predetermined annealing condition or stringent condition and is intended to encompass "substantially complementary" and "perfectly complementary", particularly, perfectly complementary.

The term "substantially complementary" means that there may be 1-4 mismatches, 1-3 mismatches, or 1-2 mismatches between two oligonucleotides.

When referring to hybridization between a portion of one oligonucleotide and entirety of another oligonucleotide, the portion of one oligonucleotide may be regarded as an individual oligonucleotide.

The hybridization may occur between two nucleic acid sequences which are perfectly complementary (perfect matched) to each other or substantially complementary even in the presence of some mismatches (e.g., 1-4 mismatches, 1-3 mismatches or 1-2 mismatches) at a hybridization occurrence site (double-strand formation site) therebetween. The degree of complementarity required for hybridization may vary depending on the hybridization reaction conditions and may be controlled by, particularly, temperature.

By way of example, the degree of complementarity required for hybridization may be 70% or higher, 75% or higher, 80% or higher, 85% or higher, 90% or higher, or 95% or higher.

According to an embodiment of the present disclosure, the degree of complementarity or the number of mismatches between two oligonucleotides is determined with reference to a site where hybridization occurs.

In the present disclosure, the suitable hybridization conditions or stringent conditions may be routinely determined by optimization procedures. Such procedures are routinely conducted by those skilled in the art to establish protocols for use in a laboratory. For example, conditions such as temperature, concentration of components, hybridization and washing times, buffer components, and their pH and ionic strength may be varied depending on various factors such as the length and GC content of oligonucleotide and target nucleotide sequence. For detailed conditions of hybridization, reference may be made to Joseph Sambrook, et al., Molecular Cloning, A Laboratory Manual, Cold Spring Harbor Laboratory Press, Cold Spring Harbor, N.Y. (2001); and M.L.M. Anderson, Nucleic Acid Hybridization, Springer-Verlag New York Inc. N.Y. (1999).

As used herein, the terms "hybridization" and "annealing" are not different from each other, and are used interchangeably with each other.

The term "sample", as used herein, is intended to encompass biological samples (e.g., cells, tissues and body fluids) and non-biological samples (e.g., food, water, and soil). The biological samples include viruses, bacteria, tissues, cells, blood (inclusive of whole blood, plasma, and serum), lymph, bone marrow fluid, saliva, sputum, swab, aspiration, milk, urine, feces, ocular fluid, semen, brain extracts, spinal cord fluid, joint fluid, thymic fluid, bronchoalveolar lavage fluid, amniotic fluid, and ascetic fluid.

According to an embodiment, the sample may be a specimen. The specimen may be obtained from a human or animal subject and may include, for example, a swab, saliva, or a mixture thereof. In detail, the swab may be a nasopharyngeal swab, a nasal swab, an oropharyngeal swab, a saliva swab, or a combination thereof.

According to an embodiment, the sample may be subjected to nucleic acid extraction and/or purification procedures for efficient amplification reactions well known in the art (see: Sambrook, J. et al., Molecular Cloning. A Laboratory Manual, 3rd ed. Cold Spring Harbor Press (2001)). The procedure of nucleic acid extraction and/or purification may depend on the types of samples.

According to an embodiment, the sample may be directly analyzed without subjecting to nucleic acid extraction or purification. For instance, use may be made of the method disclosed in Pannacio et al. (Nucleic Acids Res. 21(19): 4656, 1993), and Pandori et al. (BMC Infect Dis. 6: 104, 2006).

According to an embodiment, the sample may be a crude extract sample which has not undergone the purification of nucleic acid. For example, the method disclosed in WO2021/261864 may be employed. In detail, according to the method disclosed in WO2021/261864, a sample (e.g., a specimen stored in a transport medium) is lysed by incubation at 95°C to 100°C for 1 min to 25 min and the lysate is mixed with a composition for detecting a target nucleic acid (e.g., composition for detecting SARS-CoV-2 mutations) to provide a preparation (i.e., crude extract sample) for detecting a target nucleic acid sequence.

According to an embodiment, the crude extract sample may be obtained by treating a sample (e.g., specimen) with proteinase K to decrease the viscosity thereof, followed by incubation at 95°C to 100°C for 1 min to 25 min.

In an embodiment, the crude extract sample may be cooled to 2°C to 10°C and then mixed with the composition for detecting a target nucleic acid after the step of incubation at 95°C to 100°C for 1 min to 25 min.

In an embodiment, the crude extract sample may be mixed at a volume ratio of 1: 1 to 1:20 with the composition for detecting a target nucleic acid, particularly at a volume ratio of 1:2 to 1: 10, and more particularly at a volume ratio of 1:2 to 1:4.

In an embodiment, the crude extract sample may be mixed with the composition for detecting a target nucleic acid, without dilution.

According to an embodiment, the crude extract sample may be diluted with water or a buffer before being mixed with the composition for detecting a target nucleic acid. The dilution weakens the inhibitory action of a reaction inhibitor which may be present in the crude extract sample (e.g., reaction inhibitor which may exist in a transport medium) when the sample is mixed and reacted with the composition for detecting a target nucleic acid.

The water for dilution may be, for example, purified water, nuclease-free water (e.g., RNase-free water), or distilled water.

The buffer for dilution may be, for example, a TE buffer.

According to an embodiment, the crude extract sample may be 1- to 10-fold diluted, particularly, 2- to 8-fold, 2- to 7-fold, 2- to 6-fold, 2- to 5-fold, or 2- to 4-fold, and more particularly 2- to 4-fold. For example, for 2-fold dilution, the crude extract sample may be mixed at a ratio of 1:1 with water (or butter).

As used herein, the term "composition for detecting SARS-CoV-2 mutations" refers to a substance comprising a combination of ingredients used to detect a nucleic acid from a sample comprising a nucleic acid molecule, for example, a nucleic acid of SARS-CoV-2 virus variants in a sample from a subject suspected of infection with SARS-CoV-2 having specific mutations. The composition for detecting SARS-CoV-2 mutations may include ingredients for reverse transcription from a target RNA of SARS-CoV-2 to a target DNA, for amplifying the reverse-transcribed target DNA, and for generating a signal from the amplified target DNA.

The composition includes a plurality of PTOs (Probing and Tagging Oligonucleotide) that are respectively hybridizable with nucleotide mutation sequences of interest located in the S gene of SARS-CoV-2. The nucleotide mutation sequence of interest encodes individually for any one amino acid mutation of (i) HV69/70 del, (ii) Y144 del, (iii) E484K, (iv) N501Y, and (v) P681H.

In an embodiment, the plurality of PTOs includes: (i) a PTO hybridizing specifically with a nucleotide mutation sequence of interest encoding for the amino acid mutation of HV69/70 del; (ii) a PTO hybridizing specifically with a nucleotide mutation sequence of interest encoding for the amino acid mutation of Y144 del; (iii) a PTO hybridizing specifically with a nucleotide mutation sequence of interest encoding for the amino acid mutation of E484K; (iv) a PTO hybridizing specifically with a nucleotide mutation sequence of interest encoding for the amino acid mutation of N501Y; and (v) a PTO hybridizing specifically with a nucleotide mutation sequence of interest encoding for the amino acid mutation of P681H.

As used herein, the term "mutation" refers to a nucleotide mutation or an amino acid mutation. The "nucleotide mutation" indicates a variation in the nucleotide sequence (e.g., insertion, deletion, inversion, or substitution of one or more nucleotides, for example, single nucleotide polymorphism) relative to a reference sequence (i.e., wild-type sequence or first identified sequence) while the "amino acid mutation" indicates a variation in the amino acid sequence (e.g., insertion, substitution, or deletion of one or more amino acids, for example, internal deletion or N- or C-terminal truncation) relative to a reference sequence.

In an embodiment, the reference sequence is the nucleotide sequence or the amino acid sequence of "Severe acute respiratory syndrome coronavirus 2 (SARS-CoV-2) reference genome" (RefSeq: NC_045512.2) enrolled in the NCBI. For example, as shown in Table 1, below, HV69/70 del stands for the mutation in which the histidine residue (abbreviated to the single letter "H") at position 69 and the valine residue (V) at position 70 on the reference amino acid sequence of the S protein of SARS-CoV-2 are deleted, and N501Y stands for the mutation in which the asparagine residue (N) at position 501 on the reference amino acid sequence of the S protein of SARS-CoV-2 is substituted with a tyrosine residue (Y). In addition, the nucleotide mutation of interest encoding the mutation HV69/70 del has a deletion of the nucleotides TACATG at positions 203-208 on the reference sequence of the S gene of SARS-CoV-2, and the nucleotide mutation of interest encoding the mutation N501Y has a substitution of the nucleotide T for A at position 1,501 on the reference sequence of the S gene of SARS-CoV-2.

**[Table 1]**

| SARS-CoV-2 Mutation | | | | |
|---|---|---|---|---|
| Gene | Amino acid Mutation | Nucleotide Mutation (based on position in gene) | Nucleotide Mutation (full-genome based) | Mutated codon |
| S | HV69/70 del | 203-208 | 21765-21770 | (-TACATG) |
| | (H69del/V70 del) | | | |
| S | Y144 del | 429-431 | 21991-21993 | (-TTA) |
| S | E484K | G1450A | G23012A | (GAA->AAA) |
| S | N501Y | A1501T | A23063T | (AAT->TAT) |
| S | P681H | C2042A | C23604A | (CCT->CAT) |

The composition includes (i) a pair of primers hybridizable with a nucleotide sequence including a nucleotide mutation sequence of interest encoding for both the amino acid mutations of HV69/70 del and Y144del; (ii) a pair of primers hybridizable with a nucleic acid sequence including a nucleotide mutation sequence of interest encoding for both the amino acid mutations of E484K and N501Y; and (iii) a pair of primers hybridizable with a nucleic acid sequence including a nucleotide mutation sequence of interest encoding for the amino acid mutation P681H.

In an embodiment, the PTOs may be PTOs which hybridize with the nucleotide mutation sequence of interest in a hybridization condition, but not with a nucleotide sequence without the nucleotide mutation sequence of interest. The PTOs with such a characteristic may be more effective in detecting a deletion mutation. For example, the PTO for a nucleotide mutation sequence of interest encoding for the amino acid mutation HV69/70 del or Y144 del hybridizes with the nucleotide mutation sequence of interest, but not with a nucleotide sequence without the nucleotide mutation sequence of interest.

The PTOs include a mutation-discriminating site, that is, a site designed to discriminate a mutation. For instance, when the PTOs are designed to detect a substitution, the mutation-discriminating site possesses a complementary nucleotide to the substituted nucleotide. For a deletion, the mutation-discriminating site accounts for complementary nucleotides to the two nucleotides being adjacent to each other by the deletion.

The PTOs include a mutation-discriminating site in a middle region thereof, as defined in the claims, and as further defined herein below.

According to an embodiment, the middle region of the PTO is located apart from the 5' end thereof toward the 3' end thereof by 6 nucleotides or more, 7 nucleotides or more, 8 nucleotides or more, 9 nucleotides or more, 10 nucleotides or more, 11 nucleotides or more, or 12 nucleotides or more. According to an embodiment, the middle region is located apart from the 3' end toward the 5' end by 6 nucleotides or more, 7 nucleotides or more, 8 nucleotides or more, 9 nucleotides or more, 10 nucleotides or more, 11 nucleotides or more, or 12 nucleotides or more.

According to an embodiment, when a PTO is equally divided into three portions, the middle region is located at the second portion. According to an embodiment, when a PTO is equally divided into four portions, the middle region is located at the second and the third portion. According to an embodiment, when a PTO is equally divided into five portions, the middle region is located at the second, the third, and the fourth portions and particularly at the third portion.

According to an embodiment, the Tm value of the portion extending from the middle region to the 5' end may be substantially identical to that of the portion extending from the middle region to the 3' end in the PTO. For example, a difference in Tm value between the portion extending from the middle region to the 5' end and the portions extending from the middle region to the 3' end may be ±1°C, ±2°C, ±3°C, ±4°C, ±5°C, ±7°C, or ±9°C.

According to an embodiment, the PTO having a mutation-discriminating site located in the middle region thereof may act to hybridize with a nucleotide mutation sequence of interest in a hybridization condition, but not to hybridize with a nucleotide sequence without a nucleotide mutation sequence of interest.

According to an embodiment, the PTO may hybridize with both a nucleotide mutation sequence of interest and a nucleotide sequence without a nucleotide mutation sequence of interest in a hybridization condition, but with a difference in hybridization pattern therebetween.

The PTO with such a characteristic may be more effective for detecting a substitution mutation. For example, PTOs that are adapted for nucleotide mutation sequences of interest encoding for the amino acid mutations E484K, N501Y, and P681H, respectively, may be hybridizable with both the nucleotide mutation sequence of interest and nucleotide sequences without the nucleotide mutation sequence of interest in hybridization conditions, but a difference in hybridization pattern therebetween. Particularly there is a difference in hybridization pattern at the 5'-end region of the PTO between the nucleotide sequences.

According to an embodiment, the PTO may hybridize with both a nucleotide mutation sequence of interest and a nucleotide sequence without a nucleotide mutation sequence of interest in a hybridization, with different hybridization patterns from each other at the 5'-end region of the PTO. For example, the 5'-end region of the PTO may hybridize with a nucleotide mutation sequence of interest, but not with a nucleotide sequence without a nucleotide mutation sequence of interest.

According to an embodiment, the PTO may comprise a mutation discriminating site located apart from the 5'-end thereof by five nucleotides or less.

According to an embodiment, the mutation-discriminating site is located in the 5'-end region of the PTO. According to an embodiment, the 5'-end region of the PTO extends from the 5' end by five nucleotides or less, four nucleotides or less, three nucleotides or less, two nucleotides or less, or one nucleotide.

According to an embodiment, the PTO having a mutation-discriminating site at the 5'-end region thereof may hybridize with both a nucleotide mutation sequence of interest and a nucleotide sequence without a nucleotide mutation sequence in a hybridization condition, with different hybridization patterns therebetween at the 5'-end region of the PTO.

The PTOs include a tagging portion (e.g., 5'-tagging portion) that does not hybridize with a target nucleic acid sequence. For the PTOs, the expressions "middle region of the PTO ", and "apart from the 5' end of the PTO by five nucleotides or less" means a "middle region of a targeting region", and "apart from the 5' end of the targeting region by five nucleotides or less", respectively, wherein the targeting region is the region that excludes the tagging portion and is hybridized by the PTO.

In an embodiment, the plurality of PTOs which hybridize with nucleotide mutation sequences of interest encoding for the amino acid mutations (i) to (v), respectively, may be PTOs which hybridize with the nucleotide mutation sequence of interest in a hybridization condition, but do not with a nucleotide sequence without the nucleotide mutation sequence of interest.

In another embodiment, the plurality of PTOs which hybridize with nucleotide mutation sequences of interest encoding for the amino acid mutations (i) to (v), respectively, may be PTOs which hybridize with both the nucleotide sequences of interest and nucleotide sequences without the nucleotide sequences of interest in hybridization conditions, with a difference in hybridization pattern therebetween.

In another embodiment, some of the plurality of PTOs which hybridize with nucleotide mutation sequences of interest encoding for the amino acid mutations (i) to (v), respectively, may be PTOs which hybridize with the nucleotide mutation sequence of interest in a hybridization condition, but do not with a nucleotide sequence without the nucleotide mutation sequence of interest while the remainder may be PTOs which hybridize with both the nucleotide sequences of interest and nucleotide sequences without the nucleotide sequences of interest in hybridization conditions, with a difference in hybridization pattern therebetween.

In another embodiment, the PTOs which hybridize with nucleotide mutation sequences of interest encoding for the amino acid mutations (i) and (ii), respectively, may be PTOs which hybridize with the nucleotide mutation sequence of interest in a hybridization condition, but do not with a nucleotide sequence without the nucleotide mutation sequence of interest while the PTOs which hybridize with nucleotide mutation sequences of interest encoding for the amino acid mutations (iii) to (v), respectively, may be PTOs which hybridize with both the nucleotide sequences of interest and nucleotide sequences without the nucleotide sequences of interest in hybridization conditions, with a difference in hybridization pattern therebetween.

In another embodiment, the PTOs which hybridize with nucleotide mutation sequences of interest encoding for the amino acid mutation (ii) may be PTOs which hybridize with the nucleotide mutation sequence of interest in a hybridization condition, but do not with a nucleotide sequence without the nucleotide mutation sequence of interest while the PTOs which hybridize with nucleotide mutation sequences of interest encoding for the amino acid mutations (i), (iii), (iv) and (v), respectively, may be PTOs which hybridize with both the nucleotide sequences of interest and nucleotide sequences without the nucleotide sequences of interest in hybridization conditions, with a difference in hybridization pattern therebetween.

In a specific embodiment, the plurality of PTOs which hybridize with nucleotide mutation sequences of interest encoding for the amino acid mutations (i) to (v), respectively, are those used in the method disclosed in WO2013/133561.

In an embodiment, the incubation in step (a) may include reverse transcription and nucleic acid amplification.

According to an embodiment, the RNA virus SARS-CoV-2 may be subjected to reverse transcription before amplification of a target nucleic acid sequence. For details, reference may made to Joseph Sambrook, et al., Molecular Cloning, A Laboratory Manual, Cold Spring Harbor Laboratory Press, Cold Spring Harbor, N.Y. (2001); and Noonan, K. F. et al., Nucleic Acids Res. 16:10366 (1988).

According to a specific embodiment, the incubation may include reverse transcription PCR (RT-PCR), and more specifically one-step RT-PCR.

Reverse transcription-polymerase chain reaction (RT-PCR) is a technique combining reverse transcription of RNA into cDNA and amplification of specific DNA targets with the synthesized cDNA serving as a template.

As used herein, the term "reverse transcriptase", also known as RNA-dependent DNA polymerase, is an enzyme used to synthesize complementary DNA on an RNA template.

In an embodiment, the reverse transcriptase may be of various origins and may be, for example, avian myeloblastosis virus-derived reverse transcriptase (AMV RTase), murine leukemia virus-derived reverse transcriptase (MuLV RTase), or Rous-associated virus 2 reverse transcriptase (RAV-2 RTase), but with no limitations thereto.

A reverse transcriptase requires a primer for synthesizing cDNA from an RNA template. Primers for reverse transcription are largely divided into three types: (i) oligo-dT primer, designed to be annealed to poly-A tail to synthesize cDNA from the 3' end; (ii) random primer, a 6- to 9-mer deoxyribonucleotide mixtures comprising a random sequence to give a chance of starting the synthesis over the entire region of RNA; and (iii) target specific primer, designed to synthesize only a target cDNA.

According to an embodiment, the composition for detecting SARS-CoV-2 mutations may include a primer for reverse transcription, and the primer for reverse transcription may be a target-specific primer.

In an embodiment, the primer for reverse transcription is a target-specific primer including a complementary sequence to a target RNA and may be used to synthesize cDNA in reverse transcription and then may form a pair with another primer (e.g., forward or reverse primer) and thus act as a primer pair for amplifying the synthesized cDNA.

The amplification of a target nucleic acid molecule may be performed by various primer-involved nucleic acid amplification methods known in the art. Specifically, the amplification may be achieved according to polymerase chain reaction (PCR), which is disclosed in US Patent No. 4,683,195, No. 4,683,202, and No 4,800,159. Other examples are ligase chain reaction (LCR, US Patent Nos. 4,683,195 and 4,683,202; and PCR Protocols: A Guide to Methods and Applications (Innis et al., eds, 1990)), strand displacement amplification (SDA) (Walker, et al. Nucleic Acids Res. 20(7):1691-6 (1992); Walker PCR Methods Appl 3(1):1-6 (1993)), transcription-mediated amplification (Phyffer, et al., J. Clin. Microbiol. 34:834-841 (1996); Vuorinen, et al., J. Clin. Microbiol. 33:1856-1859 (1995)), helicase dependent amplification (HAD) (M. Vincent, Y. Xu and H. Kong, EMBO Rep., 2004, 5, 795-800), nucleic acid sequence-based amplification (NASBA) (Compton, Nature 350(6313):91-2 (1991)), rolling circle amplification (RCA) (Lisby, Mol. Biotechnol. 12(1):75-99 (1999); Hatch et al., Genet. Anal. 15(2):35-40 (1999)), Q-beta replicase (Lizardi et al., BiolTechnology 6:1197 (1988)), loop-mediated isothermal amplification (LAMP) (Y. Mori, H. Kanda and T. Notomi, J. Infect. Chemother., 2013, 19, 404-411), and recombinase polymerase amplification (RPA) (J. Li, J. Macdonald and F. von Stetten, Analyst, 2018, 144, 31-67).

Various DNA polymerases can be used for the amplification as exemplified by Klenow fragment of *E. coli* DNA polymerase I, thermostable DNA polymerase, and bacteriophage T7 DNA polymerase. In a particular embodiment, the polymerase may be any of thermostable DNA polymerases attainable from a variety of bacterial species, including *Thermus aquaticus* (Taq), *Thermusthermophilus* (Tth), *Thermus filiformis, Thermis flavus, Thermococcus literalis,* and *Pyrococcus furiosus* (Pfu). Most of the polymerases can be isolated from bacteria themselves or can be commercially available.

In an embodiment, the composition for detecting SARS-CoV-2 mutations may be provided in separated respective containers for a composition for synthesis of cDNA from RNA and a composition for cDNA amplification and detection or in a single container for a mixture thereof.

As used herein, the term "transcription" or "transcription reaction" refers to methods known in the art for enzymatically making RNA that is complementary to DNA template, thereby producing the number of RNA copies of a DNA sequence.

In the present disclosure, the composition for detecting SARS-CoV-2 mutations may optionally comprise reagents necessary for target amplification (e.g., PCR), such as a nucleic acid polymerase, a buffer, a polymerase cofactor, and deoxyribonucleotide-5-triphosphates. Optionally, the composition for detecting SARS-CoV-2 mutations may also comprise various polynucleotide molecules, a reverse transcriptase, a transcriptase, various buffers and reagents, and an antibody inhibitory of nucleic acid polymerase. In addition, the composition for detecting SARS-CoV-2 mutations may comprise an oligonucleotide or a reagent necessary for a positive control reaction. Optimal amounts of reagents to be used in a given reaction can be readily determined by the skilled artisan who understands the advantage of the present disclosure. The components of the nucleic acid amplification composition may be present in separate containers, or multiple components may be present in a single container.

According to an embodiment, the plurality of PTOs that hybridize with nucleotide mutation sequences of interest encoding for the amino acid mutations (i) to (v), respectively, may be present in a single container.

According to another embodiment, the PTOs which hybridize with nucleotide mutation sequences of interest encoding for the amino acid mutations (i) and (ii), respectively, may be present in a first container, the PTOs which hybridize with nucleotide mutation sequences of interest encoding for the amino acid mutations (iii) and (iv), respectively, may be present in a second container, and the PTOs which hybridizes with a nucleotide mutation sequence of interest encoding for the amino acid mutation (v) may be present in a third container.

In another embodiment, the PTOs which hybridize with nucleotide mutation sequences of interest encoding for the amino acid mutations (i) to (v) may be present in a first to a fifth container, respectively.

### Step (b) : Detection of SARS-CoV-2 Mutations

According to an embodiment, SARS-CoV-2 mutations can be detected by measuring signals provided depending on the presence of mutations.

According to an embodiment, step (b) may be carried out by post-amplification detection method or real-time detection method.

The post-amplification detection method is a method in which an amplicon is detected after nucleic acid amplification. Examples of the post-amplification detection method includes the separation of amplicons according to size difference (e.g., electrophoresis) or the separation of amplicons through immobilization, but is not limited thereto.

Alternatively, the post-amplification detection method may be achieved by post-PCR melting analysis wherein, after the amplification of a target nucleic acid sequence, the fluorescence intensity is monitored while the temperature is raised or lowered within a certain range, followed by detecting amplicons with reference to the melting profiles (US Patent No. 5,871,908 and No. 6,174,670, and WO 2012/096523).

The real-time detection method is designed to detect a target nucleic acid sequence while monitoring amplification of the target nucleic acid in real time. For detection of a target nucleic acid sequence, a signal can be detected at one or more temperatures during the reaction, for example, at one temperature or at two temperatures.

According to an embodiment, the real-time detection method may be carried out for steps (a) and (b) inclusive of denaturation of double-stranded nucleic acid.

The post-amplification detection method or the real-time detection method may use a label or a labeled oligonucleotide for providing a signal depending on the presence of a nucleic acid to be detected.

For example, use may be made of a non-specific fluorescence dye non-specifically intercalating into a duplex or of a labeled primer or probe.

Examples of the method using a labeled primer include the Sunrise primer method (Nazarenko et al, 2516-2521 Nucleic Acids Research, 1997, v.25 no.12, and U. S. Patent No. 6,117,635), the Scorpion primer method (Whitcombe et al., 804-807, Nature Biotechnology v.17 AUGUST 1999, and U.S. Patent No. 6,326,145), and the TSG Primer method (WO 2011/078441).

Examples of the method using a labeled probe include the molecular beacon method using a dual-labeled probe forming a hair-pin structure (Tyagi et al, Nature Biotechnology v.14 MARCH 1996), the hybridization probe method using two probes single-labeled with a donor or an acceptor (Bernad et al, 147-148 Clin Chem 2000; 46), the Lux method using a single-labeled oligonucleotide (U. S. Patent No. 7,537,886), and the TaqMan method using a cleavage reaction of the double-labeled probe by 5'- nuclease activity of DNA polymerase as well as the hybridization of a dual-labeled probe (U. S. Patent Nos. 5,210,015 and No. 5,538,848), but are not limited thereto.

In addition, the detection may be performed using a duplex formed depending on the presence of a target nucleic acid sequence. The duplex formed depending on the presence of the target nucleic acid sequence is not an amplicon itself of the target sequence formed by the amplification, but increases in quantity in proportion to the amplification of the target nucleic acid sequence. The duplex formed depending on the presence of the target nucleic acid sequence may be obtained according to various methods, for example, Invader assay (U. S. Patent Nos. 5,691,142, 6,358,691, and 6,194,149), the PTOCE (PTO Cleavage and Extension) method (WO 2012/096523), the PCEC (PTO Cleavage and Extension-dependent Cleavage) method (WO 2012/134195), the PCE-SH (PTO Cleavage and Extension-dependent Signaling Oligonucleotide Hybridization) method (WO 2013/115442), the PCE-SC (PTO Cleavage and Extension-dependent Signaling Oligonucleotide Cleavage) method (WO 2013/157821), the PCE-NH (PTO Cleavage and Extension-dependent Non-Hybridization) method (WO 2014/104818), the PCE-IH (PTO Cleavage and Extension-dependent Immobilized Oligonucleotide Hybridization) method (WO 2015/008985).

In an embodiment, step (b) may be carried out by a method for providing a signal depending on hybridization between the primer or probe and the nucleotide mutation sequence of interest.

In a particular embodiment, when the oligonucleotide that hybridizes with an nucleotide mutation sequence of interest is a probe and the probe hybridizes with the nucleotide mutation sequence of interest but not with a nucleotide sequence without the nucleotide mutation sequence of interest, use may be made of a method in which (i) the probe is linked with a dual label interactive thereto and is hybridized with the nucleotide mutation sequence of interest or (ii) the probe provides a signal in a manner dependent on the cleavage by the hybridization to the nucleotide mutation sequence of interest.

For example, a TaqMan method or a probe having a tagging portion, particularly, a 5'-tagging portion may be utilized.

The method utilizing a probe having a 5'-tagging portion may be exemplified by PTOCE (PTO Cleavage and Extension) assay (WO 2012/096523) wherein the probe having a 5'-tagging portion works as a mediation oligonucleotide and a duplex formed in a probe cleavage-dependent manner can generate a signal.

In detail, the PTOCE assay includes the following steps of:
(a) hybridizing the target nucleic acid sequence with an upstream oligonucleotide and a PTO;
(b) contacting the resulting product of step (a) with an enzyme having 5' nuclease activity in a condition for cleavage of PTO, wherein the upstream oligonucleotide or an extension strand thereof induce cleavage of the PTO by the enzyme having 5' nuclease activity, the cleavage resulting in releasing the 5'-tagging portion of the PTO or a fragment including a portion of the 5'-tagging portion;
(c) hybridizing the fragment released from the PTO with a CTO, wherein the fragment released from the PTO is hybridized with the capturing portion of the CTO;
(d) performing an extension reaction using the resulting product of step (c) and a template-dependent nucleic acid polymerase, wherein the fragment hybridized with the capturing portion of the CTO is extended to form an extended duplex and the extended duplex has a Tm value adjustable by (i) a sequence and/or length of the fragment, (ii) a sequence and/or length of the CTO, or (iii) the sequence and/or length of the fragment and the sequence and/or length of the CTO, and provides a target signal by (i) at least one label linked to the fragment and/or CTO, (ii) a label incorporated into the extended duplex during the extension reaction, (iii) at least one label linked to the fragment and/or CTO and a label incorporated into the extended duplex during the extension reaction, or (iv) an intercalating label; and
(e) detecting the extended duplex by measuring the target signal at a predetermined temperature adapted to maintain the extended duplex maintains in a double- stranded form, whereby the presence of the extended duplex indicates the presence of the target nucleic acid sequence. In this case, the method further includes repeating all or some of steps (a)-(e), with denaturation set forth between the repeating cycles.

As used herein, the term "denaturation" in the phrase "denaturation set forth between the repeating cycles" means separating a double-stranded nucleic acid molecule into a single-stranded nucleic acid molecule.

In a particular embodiment, when the oligonucleotide that hybridizes with a nucleotide mutation of interest is a probe and the probe may be hybridizable with both the nucleotide mutation of interest and a nucleotide sequence without the nucleotide mutation of interest in a hybridization condition, but with a difference in hybridization pattern therebetween, the VD-PTOCE (Variation Detection PTO Cleavage and Extension) assay (WO2013/133561) may be used for detection.

In detail, the VD-PTOCE (Variation Detection PTO Cleavage and Extension) assay includes the steps of:
(a) hybridizing the target nucleic acid sequence with an upstream oligonucleotide and a PTO-NV (Probing and Tagging Oligonucleotide for Nucleotide Variation), wherein the upstream oligonucleotide includes a hybridizing nucleotide sequence complementary to the target nucleic acid sequence and the PTO-NV includes (i) a 3'-targeting portion comprising a hybridizing nucleotide sequence complementary to the target nucleic acid sequence, (ii) a 5'-tagging portion comprising a nucleotide sequence non-complementary to the target nucleic acid sequence, and (iii) a nucleotide mutation-discriminating site, comprising a complementary sequence to the nucleotide variation on the target nucleic acid, positioned on a 5'-end part of the 3 -targeting portion, wherein the 3'-targeting portion is hybridized with the target nucleic acid sequence and the 5'-tagging portion is not hybridized with the target nucleic acid sequence, the upstream oligonucleotide is located upstream of the PTO-NV, and the upstream oligonucleotide or its extended strand induces cleavage of the PTO-NV by an enzyme having 5' nuclease activity;
(b) contacting the resulting product of step (a) with an enzyme having 5' nuclease activity in a condition for cleavage of the PTO-NV, wherein when the PTO-NV is hybridized with the target nucleic acid sequence having the nucleotide variation complementary to the nucleotide mutation-discriminating site and the 5'-end part of the 3'-targeting portion forms a double strand with the target nucleic acid sequence to induce cleavage from a first initial cleavage site, a first fragment is released, wherein when the PTO-NV is hybridized with a target nucleic acid sequence having a nucleotide variation non-complementary to the nucleotide mutation-discriminating site and the 5'-end part of the 3'-targeting portion does not form a double strand with the target nucleic acid sequence to induce cleavage from a second initial cleavage site located downstream of the first initial cleavage site, a second fragment is released, wherein the second fragment includes an additional 3'-end portion which makes the second fragment different from the first fragment;
(c) hybridizing the fragment released from the PTO-NV with a CTO (Capturing and Templating Oligonucleotide), wherein the CTO includes in the 3' to 5' direction (i) a capturing portion comprising a nucleotide sequence complementary to the 5 -tagging portion or a part of the 5'-tagging portion of the PTO-NV and (ii) a templating portion including a nucleotide sequence non-complementary to the 5'-tagging portion and the 3'-targeting portion of the PTO-NV, wherein the first fragment or the second fragment released from the PTO-NV is hybridized with the capturing portion of the CTO;
(d) performing an extension reaction using the resulting product of step (c) and a template-dependent nucleic acid polymerase, wherein when the first fragment is hybridized with the capturing portion of the CTO, it is extended to form an extended strand comprising an extended sequence complementary to the templating portion of the CTO, wherein when the second fragment is hybridized with the capturing portion of the CTO, it is not extended; and
(e) determining the presence of the extended strand, whereby the presence of the extended strand indicates the presence of the nucleotide variation complementary to the nucleotide discrimination site of the PTO-NV.

According to an embodiment, when the second fragment is hybridized with the capturing portion of the CTO, the CTO has a sequence selected to not hybridize with the additional 3'-end part of the second fragment so as to prevent the extension of the second fragment.

According to an embodiment, the nucleotide mutation-discriminating site is located apart from the 5' end of the 3'-targeting region of PTO-NV by 10 nucleotides, five nucleotides, four nucleotides, or three nucleotides or less.

According to an embodiment, the extended strand of the first fragment and the CTO form an extended duplex in the step (d); wherein the extended duplex has a Tₘ value adjustable by (i) a sequence and/or length of the first fragment, (ii) a sequence and/or length of the CTO or (iii) the sequence and/or length of the first fragment and the sequence and/or length of the CTO; wherein the extended duplex provides a target signal by (i) at least one label linked to the first fragment and/or CTO, (ii) a label incorporated into the extended duplex during the extension reaction, (iii) at least one label linked to the first fragment and/or CTO and a label incorporated into the extended duplex during the extension reaction, or (iv) an intercalating label; and wherein the presence of the extended strand is detected by measuring the target signal from the extended duplex in accordance with a melting analysis or a hybridization analysis for the extended duplex.

According to an embodiment, the extended strand of the first fragment and the CTO form an extended duplex in the step (d); wherein the extended duplex has a Tₘ value adjustable by (i) a sequence and/or length of the first fragment, (ii) a sequence and/or length of the CTO or (iii) the sequence and/or length of the first fragment and the sequence and/or length of the CTO; wherein the extended duplex provides a target signal by (i) at least one label linked to the first fragment and/or CTO, (ii) a label incorporated into the extended duplex during the extension reaction, (iii) at least one label linked to the first fragment and/or CTO and a label incorporated into the extended duplex during the extension reaction, or (iv) an intercalating label; and wherein the presence of the extended strand is detected by measuring the target signal from the extended duplex at a predetermined temperature sufficient to maintain a double strand of the extended duplex.

As used herein, the term "targeting portion" means a portion responsible for the double-stranded portion formed as a result of hybridization between a primer or a probe and a target nucleic acid sequence.

As used herein, the term "tagging portion" means a portion that is not hybridized with a target nucleic acid sequence, but remains as a single strand during the hybridization reaction between a primer or probe and the target nucleic acid sequence.

**[Table 2]**

| Mutation-discriminating site | | |
|---|---|---|
| SEQ ID NO: | Amino Acid Mutation | Sequence (5'-> 3') |
| 1 | HV69/70 del | CTCAGGACTTGTTCTTACCTTTCTTTTCCAATGTTACTTGGTT CCATGCTATCTCTGGGACCAATGGTACTAAGAGGTTTGATAA CCCTGTCCTACCATTTA |
| 2 | Y144 del | TGTTATTAAAGTCTGTGAATTTCAATTTTGTAATGATCCATT TTTGGGTGTTTACCACAAAAACAACAAAAGTTGGATGGAAAG TGAGTTCAGAGTTTATTC |
| 3 | E484K | ATATTTCAACTGAAATCTATCAGGCCGGTAGCACACCTTGT AATGGTGTTAAAGGTTTTAATTGTTACTTTCCTTTACAATCAT ATGGTTTCCAACCCACTAA |
| 4 | N501Y | AAGGTTTTAATTGTTACTTTCCTTTACAATCATATGGTTTCC AACCCACTTATGGTGTTGGTTACCAACCATACAGAGTAGTAG TACTTTCTTTTGAACTTCT |
| 5 | P681H | TACCCATTGGTGCAGGTATATGCGCTAGTTATCAGACTCAG ACTAATTCTCATCGGCGGGCACGTAGTGTAGCTAGTCAATCC ATCATTGCCTACACTATGTC |

| | | |
|---|---|---|
| **bold:** nucleotide mutation sequence of interest encoding for amino acid mutation (for deletion mutation, two nucleotides adjacent to each other as a result of deletion)underlined: nucleotide complementary to mutation-discriminating site | | |

In an embodiment, the plurality of PTOs that hybridize with the nucleotide mutation sequence of interest can hybridize with the nucleotide mutation sequence of interest of SEQ ID NOS: 1 to 5, respectively.

In a particular embodiment, the plurality of PTOs that hybridize with the nucleotide mutation sequences of interest can hybridize with complementary nucleotides (underlined) to the mutation-discriminating site of SEQ ID NOS: 1 to 5, respectively.

In a particular embodiment, a PTO that hybridizes with the nucleotide mutation sequence of interest encoding for the amino acid mutation HV 69/70 del includes a mutation-discriminating site at the middle region thereof, wherein the mutation-discriminating site can hybridize with complementary nucleotides (underlined) to the mutation-discriminating site of SEQ ID NO: 1. For example, in a PTO (or PTO-NV), the mutation-discriminating site is located in the middle region of the 3'-targeting portion of the PTO.

In a particular embodiment, a PTO that hybridizes with the nucleotide mutation sequence of interest encoding for the amino acid mutation Y144 del includes a mutation-discriminating site at the middle region thereof, wherein the mutation-discriminating site can hybridize with complementary nucleotides (underlined) to the mutation-discriminating site of SEQ ID NO: 2. For example, in a PTO (or PTO-NV), the mutation-discriminating site is located in the middle region of the 3'-targeting portion of the PTO.

In a particular embodiment, a PTO that hybridizes with the nucleotide mutation sequence of interest encoding for the amino acid mutation E484K includes a mutation-discriminating site at the 5' end thereof, wherein the mutation-discriminating site can hybridize with complementary nucleotides (underlined) to the mutation-discriminating site of SEQ ID NO: 3. For example, in a PTO (or PTO-NV), the mutation-discriminating site is located in the 5'-end region of the 3'-targeting portion of the PTO.

In a particular embodiment, a PTO that hybridizes with the nucleotide mutation sequence of interest encoding for the amino acid mutation N501Y includes a mutation-discriminating site at the 5' end or 3' end thereof, wherein the mutation-discriminating site can hybridize with complementary nucleotides (underlined) to the mutation-discriminating site of SEQ ID NO: 4. For example, in a PTO (or PTO-NV), the mutation-discriminating site is located in the 5'-end region of the 3'-targeting portion of the PTO.

In a particular embodiment, a PTO that hybridizes with the nucleotide mutation sequence of interest encoding for the amino acid mutation P681H includes a mutation-discriminating site at the 5' end or 3' end thereof, wherein the mutation-discriminating site can hybridize with complementary nucleotides (underlined) to the mutation-discriminating site of SEQ ID NO: 5. For example, in a PTO (or PTO-NV), the mutation-discriminating site is located in the 5'-end region of the 3'-targeting portion of the PTO.

In an embodiment, the plurality of PTOs that hybridize with the nucleotide mutation sequence of interest can hybridize with complementary sequence of the nucleotide mutation sequence of interest of SEQ ID NOS: 1 to 5, respectively. In this case, each of the underlined sites in Table 2 is a mutation-discrimination site in each PTO.

In an embodiment, the composition for detecting SARS-CoV-2 mutations may include a label that provides a signal depending on the presence of the target nucleic acid sequence.

In an embodiment, the label may be linked to at least one primer of the primer pair and/or to the probe.

In an embodiment, the label may be linked to at least one primer of the primer pair and/or to the probe, and can provide a signal as the primer and/or the probe hybridizes with the target nucleic acid sequence or is cleaved.

In an embodiment, the label may be linked to the probe, and can provide a signal as the probe hybridizes with the target nucleic acid sequence or is cleaved.

In an embodiment, the composition for detecting SARS-CoV-2 mutations may further include an oligonucleotide that forms a duplex, depending on the cleavage of the primer or the probe.

According to an embodiment, the label may be linked to the oligonucleotide, and can provide a signal as the oligonucleotide forms a duplex or the oligonucleotide forming a duplex is cleaved.

According to an embodiment, the label is a single label or an interactive dual label.

According to an embodiment, the label includes a fluorescent label, a luminescent label, a chemiluminescent label, and a metallic label. The label may be used by itself such as an intercalating dye. Alternatively, a single label or an interactive dual label including a donor molecule and an acceptor molecule may be used in the form bound to at least one oligonucleotide.

According to an embodiment, the single label may include a fluorescent label, a luminescent label, a chemiluminescent label, an electrochemical label, and a metal label. The single label provides different signals (e.g., different signal intensities) depending on its presence on double strand or single strand.

According to a particular embodiment, the single label may be a fluorescent label.

The single label may be linked to an oligonucleotide by various methods. By way of example, the label is linked to a probe through a spacer comprising carbon atoms (e.g., 3-carbon spacer, 6-carbon spacer, or 12-carbon spacer).

The interactive label system is a signal generating system in which energy is passed non-radioactively between a donor molecule (reporter molecule) and an acceptor molecule (quencher molecule).

As a representative interactive label system, a FRET (fluorescence resonance energy transfer) label system includes a fluorescent reporter molecule (donor molecule) and a quencher molecule (acceptor molecule). In FRET, the energy donor is fluorescent, but the energy acceptor may be fluorescent or non-fluorescent. In another form of interactive label systems, the energy donor is non-fluorescent, e.g., a chromophore, and the energy acceptor is fluorescent. In yet another form of interactive label systems, the energy donor is luminescent, e.g., bioluminescent, chemiluminescent, or electrochemiluminescent, and the acceptor is fluorescent.

An interactive dual label includes the label pair providing detectable signal based on contact-mediated quenching (Salvatore et al., Nucleic Acids Research, 2002 (30) no. 21 e122 and Johansson et al., J. AM. CHEM. SOC 2002 (124) pp 6950-6956). The interactive label system includes any or all cases in which signals are changed by interaction between at least two molecules (e.g., dyes).

The reporter and the quencher molecules useful for single or dual labels may include any molecules known in the art. Their examples include: Cy2^{™} (506), YO-PRO^{™}-1 (509), YOYO^{™}-1 (509), Calcein (517), FITC (518), FluorX^{™} (519), Alexa^{™} (520), Rhodamine 110 (520), Oregon Green^{™} 500 (522), Oregon Green^{™} 488 (524), RiboGreen^{™} (525), Rhodamine Green^{™} (527), Rhodamine 123 (529), Magnesium Green^{™}(531), Calcium Green^{™} (533), TO-PRO^{™}-1 (533), TOTO1 (533), JOE (548), BODIPY530/550 (550), Dil (565), BODIPY TMR (568), BODIPY558/568 (568), BODIPY564/570 (570), Cy3^{™} (570), Alexa^{™} 546 (570), TRITC (572), Magnesium Orange^{™} (575), Phycoerythrin R&B (575), Rhodamine Phalloidin (575), Calcium Orange^{™}(576), Pyronin Y (580), Rhodamine B (580), TAMRA (582), Rhodamine Red^{™} (590), Cy3.5^{™} (596), ROX (608), Calcium Crimson^{™} (615), Alexa^{™} 594 (615), Texas Red(615), Nile Red (628), YO-PRO^{™}-3 (631), YOYO^{™}-3 (631), R-phycocyanin (642), C-Phycocyanin (648), TO-PRO^{™}-3 (660), TOTO3 (660), DiD DilC(5) (665), Cy5^{™} (670), Thiadicarbocyanine (671), Cy5.5 (694), HEX (556), TET (536), Biosearch Blue (447), CAL Fluor Gold 540 (544), CAL Fluor Orange 560 (559), CAL Fluor Red 590 (591), CAL Fluor Red 610 (610), CAL Fluor Red 635 (637), FAM (520), Fluorescein (520), Fluorescein-C3 (520), Pulsar 650 (566), Quasar 570 (667), Quasar 670 (705), and Quasar 705 (610). The numbers in parentheses represent maximum emission wavelengths in nanometer.

Suitable pairs of reporter-quencher are disclosed in many literatures as follows: Pesce et al., editors, Fluorescence Spectroscopy (Marcel Dekker, New York, 1971); White et al., Fluorescence Analysis: A Practical Approach (Marcel Dekker, New York, 1970); Berlman, Handbook of Fluorescence Spectra of Aromatic Molecules, 2nd Edition (Academic Press, New York, 1971); Griffiths, Color AND Constitution of Organic Molecules (Academic Press, New York, 1976); Bishop, editor, Indicators (Pergamon Press, Oxford, 1972); Haugland, Handbook of Fluorescent Probes and Research Chemicals (Molecular Probes, Eugene, 1992); Pringsheim, Fluorescence and Phosphorescence (Interscience Publishers, New York, 1949); Haugland, R. P., Handbook of Fluorescent Probes and Research Chemicals, 6th Edition (Molecular Probes, Eugene, Oreg., 1996), and U. S. Patent Nos. 3,996,345 and 4,351,760.

The reporter molecules and the quencher molecules may each be fluorescent. In contrast, the reporter molecules and the quencher molecules may each be non-fluorescent. For example, a non-fluorescent dark quencher molecule capable of quenching fluorescence in a broad range of wavelengths or at a specific wavelength may be used in the present disclosure. When the quencher molecule is fluorescent, a target nucleic acid sequence can be detected from signal changes of the fluorescent quencher molecule.

The reporter molecule and the quencher molecule may be linked to oligonucleotides according to conventional methods. For example, the reporter molecule and quencher molecule may be linked to a probe through a spacer comprising at least three carbon atoms (e.g., 3-carbon spacer, 6-carbon spacer, 9-carbon spacer, or 12-carbon spacer).

As another example, signal generation by a CRISPR-based molecular diagnosis method can be utilized (Max J. et el., SHERLOCK: nucleic acid detection with CRISPR nuclease; NATURE PROTOCOLS). For instance, a signal can be generated by cleaving a labeled oligonucleotide with a CRISPR protein enzyme activated depending on the presence of a target nucleic acid sequence.

In an embodiment, the label may differ from one mutation to another.

According to an embodiment, the detection step is to determine the presence of a SARS-CoV-2 mutation when at least one of the signals for nucleotide mutations of interest encoding for the amino acid mutations (i) to (v) is detected.

In another embodiment, the labels may be the same for all mutations.

The method is conducted along with the detection of a conserved nucleic acid sequence among SARS-CoV-2 wild-type and variants, which more particularly is indicative of the presence of SARS-CoV-2 irrespective of whether the SARS-CoV-2 is a wild-type or a variant. To this end, the composition for detecting SARS-CoV-2 mutations includes an oligonucleotide capable of hybridizing with a conserved nucleic acid sequence among SARS-CoV-2 wild type and variants, in particular for amplifying and/or detecting a conserved nucleic acid sequence among SARS-CoV-2 wild-type and variants.

The detection of a conserved nucleic acid sequence among SARS-CoV-2 wild-type and variants is to determine whether SARS-CoV-2 is present or absent in a sample, but not to determine whether a specific mutation (i. e., (i) to (v) mutations) of SARS-CoV-2 is present or absent.

The composition further includes an oligonucleotide hybridizable with a conserved nucleic acid sequence among SARS-CoV-2 wild-type and variants.

In an embodiment, the conserved nucleic acid sequence among SARS-CoV-2 wild-type and variants is located at a locus selected from the group consisting of E gene, N gene, RdRP gene, and S gene of SARS-CoV-2.

As used herein, the term "oligonucleotide for amplifying and/or detecting a specific nucleic acid sequence" refers to an oligonucleotide used to amplify a specific nucleic acid sequence (e.g., target nucleic acid) and/or to detect a target nucleic acid.

According to an embodiment, the detection of a conserved nucleic acid sequence among SARS - CoV-2 wild-type and variants may be achieved by the detection of at least one of respective signals for conserved nucleic acid sequences located in E gene, RdRP gene, S gene, and N gene, which is indicative of the presence of SARS-CoV-2.

According to another embodiment, the detection of a conserved nucleic acid sequence among SARS-CoV-2 wild-type and variants may be achieved by the detection of two or more of respective signals for conserved nucleic acid sequences located in E gene, RdRP gene, S gene, and N gene, which is indicative of the presence of SARS-CoV-2.

According to a further embodiment, the detection of a conserved nucleic acid sequence among SARS-CoV-2 wild-type and variants may be achieved by the detection of three or more of respective signals for conserved nucleic acid sequences located in E gene, RdRP gene, S gene, and N gene, which is indicative of the presence of SARS-CoV-2.

According to yet another embodiment, the detection of a conserved nucleic acid sequence among SARS-CoV-2 wild-type and variants may be achieved by the detection of all of respective signals for conserved nucleic acid sequences located in E gene, RdRP gene, S gene, and N gene, which is indicative of the presence of SARS-CoV-2.

According to an embodiment, discrimination is made between a signal for detection of a SARS-CoV-2 mutation and a signal for detection of a conserved nucleic acid sequence between SARS-CoV-2 wild-type and variants (e.g., different wavelength ranges).

The method is conducted along with the detection of an internal control (IC).

The composition for detecting SARS-CoV-2 includes an oligonucleotide capable of hybridizing with a conserved nucleic acid sequence among SARS-CoV-2 wild type and variants, in particular for amplifying and/or detecting an internal control nucleic acid sequence.

The internal control may be an endogenous internal control (e.g., Human beta globin (HBB gene), RNase P, etc.) that is contained in a sample, for example, a specimen from the sampling step or may be an exogenous internal control that is added to the composition for detecting SARS-CoV-2 mutations or in the step of reacting the sample and the detecting composition.

According to an embodiment, the method may be conducted along with the detection of an endogenous internal control.

In an embodiment, the internal control may be RNase P.

According to another embodiment, the method may be conducted along with the detection of an exogenous internal control.

In an embodiment, the internal control may be a MS2 phage particle.

According to an embodiment, the method may be conducted along with the detection of an endogenous internal control and an exogenous internal control.

Provided according to another aspect is a composition for detecting SARS-CoV-2 mutations, the composition including a plurality of PTOs (Probing and Tagging Oligonucleotide) hybridizing respectively with nucleotide mutation sequences of interest located in the S gene of SARS-CoV-2,
wherein the PTO comprises (i) a 3'-targeting portion comprising a hybridizing nucleotide sequence complementary to the target nucleic acid sequence, and (ii) a 5'-tagging portion comprising a nucleotide sequence non-complementary to the target nucleic acid sequence;
wherein the nucleotide mutation sequence of interest each encodes for any one of the following amino acid mutations:
   (i) HV69/70 del,
   (ii) Y144 del,
   (iii) E484K,
   (iv) N501Y, and
   (v) P681H,
wherein (1) said PTO capable of hybridizing with the nucleotide mutation sequence of interest encoding for the amino acid mutation HV 69/70 del includes a nucleotide mutation-discriminating site at the middle region of its 3'-targeting portion, and
(2) said PTO capable of hybridizing with the nucleotide mutation sequence of interest encoding for the amino acid mutation Y144 del includes a nucleotide mutation-discriminating site at the middle region of its 3'-targeting portion;
wherein (1) said PTO capable of hybridizing with the nucleotide mutation sequence of interest encoding for the amino acid mutation E484K includes a nucleotide mutation-discriminating site in a 5'-end region of its 3'-targeting portion,
(2) said PTO capable of hybridizing with the nucleotide mutation sequence of interest encoding for the amino acid mutation N501Y includes a nucleotide mutation-discriminating site in a 5'-end region of its 3'-targeting portion, and
(3) said PTO capable of hybridizing with the nucleotide mutation sequence of interest encoding for the amino acid mutation P681H includes a nucleotide mutation-discriminating site in a 5'-end region of its 3'-targeting portion,
wherein the nucleotide mutation-discriminating site comprises a complementary sequence to the nucleotide variation on the target nucleic acid,
wherein the composition further comprises (i) a pair of primers capable of hybridizing with a nucleic acid sequence comprising the nucleotide mutation sequences of interest encoding respectively for the amino acid mutations HV69/70 del and Y144 del, (ii) a pair of primers capable of hybridizing with a nucleic acid sequence comprising the nucleotide mutation sequences of interest encoding respectively for the amino acid mutations E484K and N501Y, and (iii) a pair of primers capable of hybridizing with a nucleic acid sequence comprising the nucleotide mutation sequence of interest encoding for the amino acid mutation P681H,
wherein the composition further comprises an oligonucleotide capable of hybridizing with a conserved nucleic acid sequence among SARS-CoV-2 wild type and variants and an oligonucleotide capable of hybridizing with a nucleic acid sequence of an internal control.

Here, the "composition for detecting SARS-CoV-2 mutations" is used to conduct the "method for detecting SARS-CoV-2 mutations" of the present disclosure and thus, a description of common contents therebetween is omitted so as to avoid excessive complexity of the specification.

In an embodiment, the PTOs may hybridize with the nucleotide mutation sequence of interest, but not with a nucleotide sequence without the nucleotide mutation sequence of interest.

The PTOs include a mutation-discriminating site located in a middle region thereof, as defined in the claims, and as further defined herein above.

In an embodiment, the PTO may hybridize with both the nucleotide mutation sequence of interest and a nucleotide sequence without the nucleotide mutation sequence of interest in a hybridization condition, but with a difference in hybridization pattern therebetween.

In an embodiment, the PTO may hybridize with both the nucleotide mutation sequence of interest and a nucleotide sequence without the nucleotide mutation sequence of interest in a hybridization condition, with different hybridization patterns therebetween at the 5'-end region of the PTO.

In an embodiment, the PTO may include a mutation-discriminating site located apart from the 5' end of the PTO by five nucleotides or less.

The composition further includes (i) a pair of primers capable of hybridizing with a nucleic acid sequence comprising nucleotide mutation sequences of interest encoding respectively for the amino acid mutations HV69/70 del and Y144 del; (ii) a pair of primers capable of hybridizing with a nucleic acid sequence comprising nucleotide mutation sequences of interest encoding respectively for the amino acid mutations E484K and N501Y; and (iii) a pair of primers capable of hybridizing with a nucleic acid sequence comprising a nucleotide mutation sequence of interest encoding for the amino acid mutation P681H.

The composition further includes an oligonucleotide capable of hybridizing with a conserved nucleic acid sequence among SARS-CoV-2 wild type and variants.

In an embodiment, the conserved nucleic acid sequence is located in a nucleic acid sequence selected from the group consisting of E gene, N gene, RdRP gene, and S gene.

The composition further includes an oligonucleotide capable of hybridizing with a nucleic acid sequence of an internal control.

In an embodiment, the internal control is RNase P.

### [Mode for Invention]

A better understanding of the present disclosure may be obtained through the following examples which are set forth to illustrate, but are not to construed as limiting the present disclosure.

### EXAMPLES

It was confirmed that nucleotide mutations of interest encoding the amino acid mutations HV69/70del, Y144del, E484K, N501Y, and P681H of SARS-CoV-2 can be detected according to the method of the present disclosure.

### EXAMPLE 1. Preparation of Sample

### 1-1. Preparation of RNA template comprising single mutation

Samples comprising nucleic acid molecules were prepared. To this end, RNAs comprising nucleotide mutation sequences of interest (Table 2) encoding for the amino acid mutations HV69/70del, Y144del, E484K, N501Y, and P681H located in the spike (S) gene, respectively, were used as templates for SARS-CoV-2 mutations. To attain the templates, the synthesis of plasmid DNAs into which sequences of the S gene comprising nucleotide sequences of interest encoding for the amino acid mutations HV69/70del, Y144del, E484K, N501Y, and P681H, respectively, were cloned, together with a T7 promoter sequence, was entrusted to IDT (Integrated DNA Technologies, USA). The synthesized plasmid DNAs were subjected to in vitro transcription using T7 RNA polymerase to afford RNAs comprising the five mutations respectively.

As target sequences for detection of conserved nucleic acid sequences among SARS-CoV-2 wild-type and variants, RNAs of the conserved nucleic acid sequences located in E gene, RdRP gene, S gene, and N gene of SARS-CoV-2 were used. In this regard, the synthesis of plasmid DNAs carrying respective gene sequences and a T7 promoter sequence was entrusted to IDT. Thereafter, the synthesized plasmid DNAs were subjected to in vitro transcription using T7 RNA polymerase to prepare an E gene template, an RdRP gene template, an S gene template, and an N gene template.

### 1-2. Preparation of RNA template comprising multiple mutations

Two RNAs corresponding to the lists of Table 3, below, were purchased from the collection culture TWIST BIOSCIENCE.

**[Table 3]**

| N o. | Organism | Source | Isolate No (Cat no.) | Mutation | Status |
|---|---|---|---|---|---|
| 1 | SARS-CoV-2 variant B.1.1.7 | TWIST BIOSCIENCE | Control 14 Cat no. 103907 | HV69/70del, Y144del, N501Y, P681H | Synthetic RNA |

| No. | Organism | Source | Isolate No (Cat no.) | Mutation | Status |
|---|---|---|---|---|---|
| 2 | SARS-CoV-2 variantB.1.351 | TWIST BIOSCIENCE | Control 16 Cat no. 104043 | N501Y, E484K | Synthetic RNA |

The two synthetic RNAs purchased from the culture collection provide a coverage of 99.9% for the viral genome of each variant. SARS-CoV-2 variant B.1.1.7 (Cat No. 103907, TWIST BIOSCIENCE) included nucleotide mutation sequences of interest encoding for the amino acid mutations HV69/70del, Y144del, N501Y, and P681H while SARS-CoV-2 variant B.1.351 (Cat No. 104043, TWIST BIOSCIENCE) included nucleotide mutation sequences of interest encoding for the amino acid mutations E484K and N501Y.

In addition, SARS-CoV-2 variant B.1.1.7 (Cat No. 103907, TWIST BIOSCIENCE) and SARS-CoV-2 variantB.1.351 (Cat No. 104043, TWIST BIOSCIENCE) each included conserved nucleic acid sequences located E gene, RdRP gene, S gene, and N gene among SARS-CoV-2 wild-type and variants.

### 1-3. Preparation of swab specimen and saliva specimen

### (1) Preparation of swab specimen with RNA template comprising single mutation

The nucleotide mutation sequence of interest, prepared in Example 1-1, encoding for the amino acid mutations HV69/70del, Y144del, E484K, N501Y, and P681H were added to a negative swab specimen to give six types of swab specimens, as shown in Table 4, each having a total of three concentrations (concentration 1: 10⁶ copies/mL, concentration 2: 10⁵ copies/mL, concentration 3: 10⁴ copies/mL).

For the negative clinical specimen, a nasopharyngeal swab and an oropharyngeal (throat) swab were taken using a specimen sampling kit, pooled in one UTM (Universal Transport Medium, COPAN, 360C), and stored at -80°C before use.

**[Table 4]**

| Sample | | | | | | |
|---|---|---|---|---|---|---|
| Mutation | Sample No. | | | | | |
| | 1 | 2 | 3 | 4 | 5 | 6 |
| HV69/70del | - | + | - | - | - | - |
| Y144del | - | - | + | - | - | - |
| E484K | - | - | - | + | - | - |
| N501Y | - | - | - | - | + | - |
| P681H | - | - | - | - | - | + |

| | | | | | | |
|---|---|---|---|---|---|---|
| +: presence; -: absence | | | | | | |

Subsequently, an extraction kit (STARMag 96 X 4 Universal Cartridge Kit, Seegene) and 300 µL of each of the prepared swab specimens were mounted to an automated extraction system (Microlab NIMBUS IVD, Hamilton) and nucleic acid extraction was conducted with an elution volume of 100 µL to obtain nucleic acid extracts of the swab specimens.

### (2) Preparation of swab specimen with RNA template comprising multiple mutations

The two RNA templates prepared in Example 1-2 were diluted with a negative swab specimen to prepare various concentrations of swab specimens as listed in Table 5.

**[Table 5]**

| Sample | | |
|---|---|---|
| Conc. (copies/mL) | SARS-CoV-2 B.1.1.7 RNA | SARS-CoV-2 B.1.351 RNA |
| 1 | 3.2X10^5 | 2X10^5 |
| 2 | 8X10^4 | 5X10^4 |
| 3 | 2X10^4 | 1.25X10^4 |
| 4 | 5X10^3 | 3.125X10^3 |

Subsequently, 15 µl of each of the prepared swab specimens (i.e., transport medium comprising the specimen) was diluted with 45 µl of distilled water, and the dilutions were incubated at 98°C for 3 min and cooled at 4°C for 5 min in a thermal cycler to prepare crude extract samples of the swab specimens.

### (3) Preparation of saliva specimen with RNA template comprising multiple mutations

The two RNA templates prepared in Example 1-2 were diluted with a negative saliva specimen to prepare saliva specimens at various concentrations as shown in Table 6.

**[Table 6]**

| Sample | | | | |
|---|---|---|---|---|
| Conc. (copies/mL) | For nucleic acid extraction | | For crude extract | |
| | SARS-CoV-2 B.1.1.7 RNA | SARS-CoV-2 B.1.351 RNA | SARS-CoV-2 B.1.1.7 RNA | SARS-CoV-2 B.1.351 RNA |
| 1 | 5 X10^3 | 5 X10^3 | 3.2X10^5 | 2X10^5 |
| 2 | 1.5 X10^3 | 1.5 X10^3 | 8X10^4 | 5X10^4 |
| 3 | - | - | 2X10^4 | 1.25X10^4 |
| 4 | - | - | 5X10^3 | 3.125X10^3 |

The negative saliva specimen was taken into a blank tube without bubbles from a person who had neither ingested anything including water, nor smoked and brushed the teeth from one hour before specimen sampling. The specimen was stored at -20°C before use.

### (i) Preparation of saliva nucleic acid extract

An extraction kit (STARMag 96 X 4 Universal Cartridge Kit, Seegene) and 300 µL of each of the prepared saliva specimens were mounted to an automated extraction system (Microlab NIMBUS IVD, Hamilton) and nucleic acid extraction was conducted with an elution volume of 100 µL to obtain nucleic acid extracts of the saliva specimens.

### (ii) Preparation saliva crude extract

In each tube, 10 µl of the saliva specimen, 30 µl of 1x TE buffer (1 mM EDTA, 10 mM Tris-HCl (pH 8.0)), and 5 µl of proteinase K were mixed to give a total volume of 45 µl comprising protease K at a concentration of 2.22 mg/mL. Then, the dilutions were incubated at 98°C for 3 min and cooled at 4°C for 5 min in a thermal cycler to prepare crude extract samples of the saliva specimen.

### EXAMPLE 2. Preparation of Composition for Detecting SARS-CoV-2 Mutations

Used for detection of the target nucleic acids were TaqMan real-time PCR (U. S. Patent Nos. 5,210,015 and 5,538,848), and PTOCE assay (WO 2012/096523) and VD-PTOCE assay (WO 2013/133561), which can detect a plurality of targets by using signals from duplexes formed depending on the presence of target nucleic acid sequences. For extension of forward and reverse primers and cleavage of probes, TaqDNA polymerase with 5' nuclease activity was employed.

As an endogenous internal control, RNase P was used.

First, oligonucleotides for detecting SARS-CoV-2 mutations which comprise five PTOs (or PTO-NVs) that are oligonucleotides hybridizing respectively with five nucleotide mutation sequences of interest in Table 2 and three primer pairs for amplifying a nucleic acid sequence comprising the nucleotide mutation sequences of Table 2; a primer pairs for amplification and detection of RNase P; and an oligonucleotide, inclusive of a TaqMan probe, for detecting the internal control were prepared as follows. PTO for detecting the amino acid mutation Y144 del was designed to have mutation-discriminating site located in the middle region of the 3'-targeting portion thereof while PTOs for detecting the amino acid mutations HV69/70 del, E484K, N501Y, and P681H were designed to have mutation-discriminating site located in the 5' end region of the 3'-targeting portion thereof. In addition, CTOs for detecting nucleotide mutation sequences of interest were designed to have the same label (Q705) (that is to say, detected in the same channel). The TaqMan probe for detecting RNase P was labeled with a fluorescent reporter (HEX) at the 5' end thereof and with a quencher molecule at the 3' end thereof.

A 4x oligonucleotide mixture for SARS-CoV-2 mutations was prepared to contain a pair of primers for amplifying a region including nucleotide mutations of interest encoding for the amino acid mutations HV69/70 del and Y144 del, a pair of primers for amplifying a region including nucleotide mutations of interest encoding for the amino acid mutations E484K and N501Y, a pair of primers for amplifying a region including a nucleotide mutation of interest encoding for the amino acid mutation P681H, and a pair of primers for amplifying RNase P at a concentration of 0.25 µM for each pair, a probe for detecting RNase P at a concentration of 0.1 µM, and PTOs and CTOs for detecting nucleotide mutations of interest encoding for the amino acid mutations HV69/70 del, Y144 del, E484K, N501Y, and P681H at a concentration of 0.5 µM for each PTO and 0.05 µM for each CTO.

Used as oligonucleotides for detecting conserved nucleic acid sequences among SARS-CoV-2 wild-type and variants was SARS2 MOM, which is a component of the multiplex one-step RT-PCR product Allplex^{™} SARS-CoV-2 Assay (Cat.No.RV10248X, Seegene Inc.), which can detect multiple targets in a single tube.

In addition, enzymes were contained in EM8, which is a component of Allplex^{™} SARS-CoV-2 Assay (Cat.No.RV10248X, Seegene Inc.). EM8 contains reagents, UDG, and dNTP necessary for reverse transcription and amplification as well as reverse transcriptase and Taq DNA polymerase.

Finally, the composition for detecting SARS-CoV-2 mutation was prepared by mixing 5 µL of the 4x oligonucleotide mixture for detecting SARS-CoV-2 mutation with 5 µL of SARS2 MOM and 5µL of EM8, which are both components of Allplex^{™} SARS-CoV-2 Assay (Cat.No. RV10248X, Seegene Inc.).

### EXAMPLE 3. One-step RT-PCR

To 5 µL of the crude extract sample or nucleic acid extract sample of the swab specimen or the crude extract sample or nucleic acid extract sample of the saliva specimen, prepared in Example 1, was added 15 µL of the composition for detecting SARS-CoV-2 mutations, prepared in Example 2, to give a total of 20 µL of a reaction mixture.

After being placed in a real-time thermal cycler (CFX96, Bio-Rad), each tube comprising the prepared reaction mixture was incubated at 50°C for 20 min, denatured at 95°C for 15 min, and then subjected to 45 cycles of 95°C for 10 sec, 60°C for 15 sec, and 72°C for 10 sec. Signal detection was conducted at 60°C every cycle. Signals detected at the temperature were analyzed using the analysis program Seegene Viewer software. For every preparation, the experiment was performed in duplicate. Mean Ct values were obtained from measurements of the repeated experiments.

As a result, it was confirmed that the method according to the present disclosure could detect the five SARS-CoV-2 mutations as demonstrated from the data of Tables 7 to 10.

In addition, it was observed that conserved nucleic acid sequences among SARS-CoV-2 wild-type and variants could be detected along with detection of the five mutations.

Particularly, the five SARS-CoV-2 mutations could be detected from the crude extract samples as well as nucleic acid extracts.

**[Table 7]**

| Mean Ct value for Nucleic Acid Extract Sample of Swab Specimen | | | |
|---|---|---|---|
| No. | Mutation | Conc. (copies/mL) | Mean Ct |
| 1. | Negative control | - | - |
| 2. | H69/70d | 10^6 | 27.71 |
| | | 10^5 | 31.23 |
| | | 10^4 | 34.63 |
| 3. | Y144d | 10^6 | 27.46 |
| | | 10^5 | 30.86 |
| | | 10^4 | 34.00 |
| 4. | E484K | 10^6 | 28.77 |
| | | 10^5 | 32.13 |
| | | 10^4 | 35.88 |
| 5. | N501Y | 10^6 | 27.53 |
| | | 10^5 | 31.21 |
| | | 10^4 | 34.32 |
| 6. | P681H | 10^6 | 27.70 |
| | | 10^5 | 31.52 |
| | | 10^4 | 35.74 |

**[Table 8]**

| Mean Ct value for Crude Extract Sample of Swab Specimen | | | | | | |
|---|---|---|---|---|---|---|
| Temple | Conc (copies/mL) | Gene | | | | |
| | | E (FAM) | Rd RP/S (C6 10) | N (Q670 ) | S mutation (Q705) | END O IC (HE X) |
| SARS-CoV-2 B.1.1.7 RNA | 3.2X10^5 | 31.49 | 32.5 1 | 33.49 | 31.54 | 25.8 9 |
| | 8X10^4 | 33.67 | 34.6 6 | 35.27 | 34.11 | 25.9 5 |
| | 2X10^4 | 35.64 | 36.9 1 | 37.27 | 36.73 | 25.9 7 |
| | 5X10^3 | 37.12 | 39.3 4 | 38.20 | 37.38 | 26.0 7 |
| SARS-CoV-2 B.1.351 RNA | 2X10^5 | 31.14 | 32.3 1 | 32.39 | 33.06 | 26.8 3 |
| | 5X10^4 | 33.38 | 34.0 9 | 34.35 | 35.27 | 26.7 7 |
| | 1.25X10^4 | 35.55 | 36.6 6 | 36.16 | 37.21 | 26.7 5 |
| | 3.125X10^3 | 37.15 | 39.6 3 | 37.21 | 37.49 | 26.7 8 |

**[Table 9]**

| Mean Ct value for Nucleic acid Extract Sample of Saliva Specimen | | | | | | |
|---|---|---|---|---|---|---|
| Target | Conc. (copies/mL) | Gene | | | | |
| | | E (FA M) | RdRP/S (C610) | N (Q670 ) | S mutation (Q705) | END O IC (HE X) |
| SARS-CoV-2 B.1.1.7 RNA | 5X10^3 | 33.1 4 | 33.37 | 34.59 | 33.69 | 22.4 5 |
| | 1.5X10^3 | 36.1 5 | 36.55 | 37.10 | 36.88 | 22.4 3 |
| SARS-CoV-2 B.1.351 RNA | 5X10^3 | 34.5 6 | 35.65 | 36.02 | 36.66 | 22.0 3 |
| | 1.5X10^3 | 37.5 1 | 38.73 | 37.76 | 37.15 | 22.3 3 |

**[Table 10]**

| Mean Ct value for Crude Extract Sample of Saliva Specimen | | | | | | |
|---|---|---|---|---|---|---|
| Temple | Conc. (copies/mL) | Gene | | | | |
| | | E (FA M) | RdRP /S (C610 ) | N (Q670 ) | S mutation (Q705) | END O IC (HE X) |
| SARS-CoV-2 | 3.2X10^5 | 31.6 5 | 33.47 | 33.74 | 33.37 | 29.6 0 |
| B.1.1.7 RNA | 8X10^4 | 33.5 1 | 35.09 | 35.23 | 35.23 | 29.3 7 |
| | 2X10^4 | 36.0 9 | 37.01 | 37.82 | 37.04 | 29.2 2 |
| | 5X10^3 | 36.7 7 | 38.20 | 36.55 | 37.67 | 29.1 7 |
| SARS-CoV-2 B.1.351 RNA | 2X10^5 | 30.8 2 | 32.32 | 32.93 | 33.26 | 31.1 9 |
| | 5X10^4 | 33.6 1 | 34.73 | 35.19 | 36.30 | 31.2 9 |
| | 1.25X10^4 | 35.0 4 | 37.13 | 36.98 | 37.26 | 31.5 2 |
| | 3.125X10^3 | 36.8 5 | 37.84 | 37.72 | 37.62 | 30.7 5 |

## Claims

1. A method for detecting SARS-CoV-2 mutations in a sample comprising a nucleic acid molecule, the method comprising the steps of:
(a) incubating the sample with a composition for detecting SARS-CoV-2 mutations; and
(b) detecting the SARS-CoV-2 mutations,
wherein the composition comprises a plurality of PTOs (Probing and Tagging Oligonucleotide) hybridizing respectively with nucleotide mutation sequences of interest located in the S gene of SARS-CoV-2,
wherein the PTO comprises (i) a 3'-targeting portion comprising a hybridizing nucleotide sequence complementary to the target nucleic acid sequence, and (ii) a 5'-tagging portion comprising a nucleotide sequence non-complementary to the target nucleic acid sequence;
wherein the nucleotide mutation sequences of interest each encodes for any one of the following amino acid mutations:
(i) HV69/70 del,
(ii) Y144 del,
(iii) E484K,
(iv) N501Y, and
(v) P681H,
wherein (1) said PTO capable of hybridizing with the nucleotide mutation sequence of interest encoding for the amino acid mutation HV 69/70 del includes a nucleotide mutation-discriminating site at the middle region of its 3'-targeting portion, and
(2) said PTO capable of hybridizing with the nucleotide mutation sequence of interest encoding for the amino acid mutation Y144 del includes a nucleotide mutation-discriminating site at the middle region of its 3'-targeting portion;
wherein (1) said PTO capable of hybridizing with the nucleotide mutation sequence of interest encoding for the amino acid mutation E484K includes a nucleotide mutation-discriminating site in a 5'-end region of its 3'-targeting portion,
(2) said PTO capable of hybridizing with the nucleotide mutation sequence of interest encoding for the amino acid mutation N501Y includes a nucleotide mutation-discriminating site in a 5'-end region of its 3'-targeting portion, and
(3) said PTO capable of hybridizing with the nucleotide mutation sequence of interest encoding for the amino acid mutation P681H includes a nucleotide mutation-discriminating site in a 5'-end region of its 3'-targeting portion,
wherein the nucleotide mutation-discriminating site comprises a complementary sequence to the nucleotide variation on the target nucleic acid,
wherein the composition further comprises (i) a pair of primers capable of hybridizing with a nucleic acid sequence comprising the nucleotide mutation sequences of interest encoding respectively for the amino acid mutations HV69/70 del and Y144 del, (ii) a pair of primers capable of hybridizing with a nucleic acid sequence comprising the nucleotide mutation sequences of interest encoding respectively for the amino acid mutations E484K and N501Y, and (iii) a pair of primers capable of hybridizing with a nucleic acid sequence comprising the nucleotide mutation sequence of interest encoding for the amino acid mutation P681H,
wherein the composition further comprises an oligonucleotide capable of hybridizing with a conserved nucleic acid sequence among SARS-CoV-2 wild type and variants and an oligonucleotide capable of hybridizing with a nucleic acid sequence of an internal control.

2. The method of claim 1, wherein the sample is a swab, saliva, or a combination thereof; wherein preferably the swab is a nasopharyngeal swab, a nasal swab, an oropharyngeal swab, a saliva swab, or a combination thereof.

3. The method of claim 1, wherein the sample is a crude extract sample which has not been subjected to any purification of a nucleic acid sequence.

4. The method of claim 1, wherein the incubation comprises a reverse transcription and a nucleic acid amplification reaction.

5. The method of claim 1, each PTO capable of hybridizing with the nucleotide mutation sequence of interest encoding for the amino acid mutations E484K, N501Y and P681H, comprises a nucleotide mutation-discriminating site located apart from the 5'-end of its 3'-targeting portion by 5 nucleotides or less.

6. The method of claim 1, wherein the conserved nucleic acid sequence among SARS-CoV-2 wild type and variants is located in a nucleic acid sequence selected from the group consisting of E gene, N gene, RdRP gene, and S gene of SARS-COV-2.

7. The method of claim 1, wherein the internal control is RNase P.

8. A composition for detecting SARS-CoV-2 mutations, the composition comprising a plurality of PTOs (Probing and Tagging Oligonucleotide) hybridizing respectively with nucleotide mutation sequences of interest located in the S gene of SARS-CoV-2,
wherein the PTO comprises (i) a 3'-targeting portion comprising a hybridizing nucleotide sequence complementary to the target nucleic acid sequence, and (ii) a 5'-tagging portion comprising a nucleotide sequence non-complementary to the target nucleic acid sequence;
wherein the nucleotide mutation sequences of interest each encodes for any one of the following amino acid mutations:
(i) HV69/70 del,
(ii) Y144 del,
(iii) E484K,
(iv) N501Y, and
(v) P681H,
wherein (1) said PTO capable of hybridizing with the nucleotide mutation sequence of interest encoding for the amino acid mutation HV 69/70 del includes a nucleotide mutation-discriminating site at the middle region of its 3'-targeting portion, and
(2) said PTO capable of hybridizing with the nucleotide mutation sequence of interest encoding for the amino acid mutation Y144 del includes a nucleotide mutation-discriminating site at the middle region of its 3'-targeting portion;
wherein (1) said PTO capable of hybridizing with the nucleotide mutation sequence of interest encoding for the amino acid mutation E484K includes a nucleotide mutation-discriminating site in a 5'-end region of its 3'-targeting portion,
(2) said PTO capable of hybridizing with the nucleotide mutation sequence of interest encoding for the amino acid mutation N501Y includes a nucleotide mutation-discriminating site in a 5'-end region of its 3'-targeting portion, and
(3) said PTO capable of hybridizing with the nucleotide mutation sequence of interest encoding for the amino acid mutation P681H includes a nucleotide mutation-discriminating site in a 5'-end region of its 3'-targeting portion,
wherein the nucleotide mutation-discriminating site comprises a complementary sequence to the nucleotide variation on the target nucleic acid,
wherein the composition further comprises (i) a pair of primers capable of hybridizing with a nucleic acid sequence comprising the nucleotide mutation sequences of interest encoding respectively for the amino acid mutations HV69/70 del and Y144 del, (ii) a pair of primers capable of hybridizing with a nucleic acid sequence comprising the nucleotide mutation sequences of interest encoding respectively for the amino acid mutations E484K and N501Y, and (iii) a pair of primers capable of hybridizing with a nucleic acid sequence comprising the nucleotide mutation sequence of interest encoding for the amino acid mutation P681H,
wherein the composition further comprises an oligonucleotide capable of hybridizing with a conserved nucleic acid sequence among SARS-CoV-2 wild type and variants and an oligonucleotide capable of hybridizing with a nucleic acid sequence of an internal control.

9. The composition of claim 8, wherein each PTO capable of hybridizing with the nucleotide mutation sequence of interest encoding for the amino acid mutations E484K, N501Y and P681H, comprises a nucleotide mutation-discriminating site located apart from the 5'-end of its 3'-targeting portion by 5 nucleotides or less.

10. The composition of claim 8, wherein the conserved nucleic acid sequence among SARS-CoV-2 wild type and variants is located in a nucleic acid sequence selected from the group consisting of E gene, N gene, RdRP gene, and S gene of SARS-COV-2.

11. The composition of claim 8, wherein the internal control is RNase P.

## Patentansprüche

1. Verfahren zum Nachweis von SARS-CoV-2-Mutationen in einer Probe, die ein Nukleinsäuremolekül umfasst, wobei das Verfahren die folgenden Schritte umfasst:
(a) Inkubieren der Probe mit einer Zusammensetzung zum Detektieren von SARS-CoV-2-Mutationen; und
(b) Detektieren der SARS-CoV-2-Mutationen,
wobei die Zusammensetzung eine Vielzahl von PTOs (Probing and Tagging Oligonucleotide; Sondierungs- und Markierungsoligonukleotid) umfasst, die jeweils mit Nukleotidmutationssequenzen von Interesse hybridisieren, die sich im S-Gen von SARS-CoV-2 befinden,
wobei das PTO (i) einen 3'-Targeting-Abschnitt, der eine hybridisierende Nukleotidsequenz umfasst, die zur Zielnukleinsäuresequenz komplementär ist, und (ii) einen 5'-Markierungsabschnitt, der eine Nukleotidsequenz umfasst, die zur Zielnukleinsäuresequenz nicht komplementär ist, umfasst;
wobei die Nukleotidmutationssequenzen von Interesse jeweils für eine der folgenden Aminosäuremutationen kodieren:
(i) HV69/70 del,
(ii) Y144 del,
(iii) E484K,
(iv) N501Y und
(v) P681H,
wobei (1) das PTO, das mit der Nukleotidmutationssequenz von Interesse hybridisieren kann, die für die Aminosäuremutation HV 69/70 del kodiert, eine Nukleotidmutationsunterscheidungsstelle in der mittleren Region seines 3'-Targeting-Abschnitts einschließt und
(2) das PTO, das mit der Nukleotidmutationssequenz von Interesse hybridisieren kann, die für die Aminosäuremutation Y144 del kodiert, eine Nukleotidmutationsunterscheidungsstelle in der mittleren Region seines 3'-Targeting-Abschnitts einschließt;
wobei (1) das PTO, das mit der Nukleotidmutationssequenz von Interesse hybridisieren kann, die für die Aminosäuremutation E484K kodiert, eine Nukleotidmutationsunterscheidungsstelle in einer 5'-Endregion seines 3'-Targeting-Abschnitts einschließt,
(2) das PTO, das mit der Nukleotidmutationssequenz von Interesse hybridisieren kann, die für die Aminosäuremutation N501Y kodiert, eine Nukleotidmutationsunterscheidungsstelle in einer 5'-Endregion seines 3'-Targeting-Abschnitts einschließt und
(3) das PTO, das mit der Nukleotidmutationssequenz von Interesse hybridisieren kann, die für die Aminosäuremutation P681H kodiert, eine Nukleotidmutationsunterscheidungsstelle in einer 5'-Endregion seines 3'-Targeting-Abschnitts einschließt,
wobei die Nukleotidmutationsunterscheidungsstelle eine zur Nukleotidvariation auf der Zielnukleinsäure komplementäre Sequenz umfasst,
wobei die Zusammensetzung ferner (i) ein Primerpaar, das mit einer Nukleinsäuresequenz hybridisieren kann, die die Nukleotidmutationssequenzen von Interesse umfasst, die jeweils für die Aminosäuremutationen HV69/70 del und Y144 del kodieren, (ii) ein Primerpaar, das mit einer Nukleinsäuresequenz hybridisieren kann, die die Nukleotidmutationssequenzen von Interesse umfasst, die jeweils für die Aminosäuremutationen E484K und N501Y kodieren, und (iii) ein Primerpaar, das mit einer Nukleinsäuresequenz hybridisieren kann, die die Nukleotidmutationssequenz von Interesse umfasst, die für die Aminosäuremutation P681H kodiert, umfasst,
wobei die Zusammensetzung ferner ein Oligonukleotid, das mit einer unter SARS-CoV-2-Wildtyp und -Varianten konservierten Nukleinsäuresequenz hybridisieren kann, und ein Oligonukleotid, das mit einer Nukleinsäuresequenz einer internen Kontrolle hybridisieren kann, umfasst.

2. Verfahren nach Anspruch 1, wobei die Probe ein Tupfer, Speichel oder eine Kombination davon ist; wobei der Tupfer vorzugsweise ein Nasopharynxtupfer, ein Nasentupfer, ein Oropharynxtupfer, ein Speicheltupfer oder eine Kombination davon ist.

3. Verfahren nach Anspruch 1, wobei die Probe eine Rohextraktprobe ist, die keiner Reinigung einer Nukleinsäuresequenz unterzogen wurde.

4. Verfahren nach Anspruch 1, wobei die Inkubation eine reverse Transkription und eine Nukleinsäureamplifikationsreaktion umfasst.

5. Verfahren nach Anspruch 1, wobei jedes PTO, das mit der Nukleotidmutationssequenz von Interesse hybridisieren kann, die für die Aminosäuremutationen E484K, N501Y und P681H kodiert, eine Nukleotidmutationsunterscheidungsstelle umfasst, die sich vom 5'-Ende seines 3'-Targeting-Abschnitts um 5 Nukleotide oder weniger entfernt befindet.

6. Verfahren nach Anspruch 1, wobei sich die unter SARS-CoV-2-Wildtyp und - Varianten konservierte Nukleinsäuresequenz in einer Nukleinsäuresequenz befindet, die aus der Gruppe bestehend aus E-Gen, N-Gen, RdRP-Gen und S-Gen von SARS-COV-2 ausgewählt ist.

7. Verfahren nach Anspruch 1, wobei die interne Kontrolle RNase P ist.

8. Zusammensetzung zum Nachweis von SARS-CoV-2-Mutationen, wobei die Zusammensetzung eine Vielzahl von PTOs (Probing and Tagging Oligonucleotide; Sondierungs- und Markierungsoligonukleotid) umfasst, die jeweils mit Nukleotidmutationssequenzen von Interesse hybridisieren, die sich im S-Gen von SARS-CoV-2 befinden,
wobei das PTO (i) einen 3'-Targeting-Abschnitt, der eine hybridisierende Nukleotidsequenz umfasst, die zur Zielnukleinsäuresequenz komplementär ist, und (ii) einen 5'-Markierungsabschnitt, der eine Nukleotidsequenz umfasst, die zur Zielnukleinsäuresequenz nicht komplementär ist, umfasst;
wobei die Nukleotidmutationssequenzen von Interesse jeweils für eine der folgenden Aminosäuremutationen kodieren:
(i) HV69/70 del,
(ii) Y144 del,
(iii) E484K,
(iv) N501Y und
(v) P681H,
wobei (1) das PTO, das mit der Nukleotidmutationssequenz von Interesse hybridisieren kann, die für die Aminosäuremutation HV 69/70 del kodiert, eine Nukleotidmutationsunterscheidungsstelle in der mittleren Region seines 3'-Targeting-Abschnitts einschließt und
(2) das PTO, das mit der Nukleotidmutationssequenz von Interesse hybridisieren kann, die für die Aminosäuremutation Y144 del kodiert, eine Nukleotidmutationsunterscheidungsstelle in der mittleren Region seines 3'-Targeting-Abschnitts einschließt;
wobei (1) das PTO, das mit der Nukleotidmutationssequenz von Interesse hybridisieren kann, die für die Aminosäuremutation E484K kodiert, eine Nukleotidmutationsunterscheidungsstelle in einer 5'-Endregion seines 3'-Targeting-Abschnitts einschließt,
(2) das PTO, das mit der Nukleotidmutationssequenz von Interesse hybridisieren kann, die für die Aminosäuremutation N501Y kodiert, eine Nukleotidmutationsunterscheidungsstelle in einer 5'-Endregion seines 3'-Targeting-Abschnitts einschließt und
(3) das PTO, das mit der Nukleotidmutationssequenz von Interesse hybridisieren kann, die für die Aminosäuremutation P681H kodiert, eine Nukleotidmutationsunterscheidungsstelle in einer 5'-Endregion seines 3'-Targeting-Abschnitts einschließt,
wobei die Nukleotidmutationsunterscheidungsstelle eine zur Nukleotidvariation auf der Zielnukleinsäure komplementäre Sequenz umfasst,
wobei die Zusammensetzung ferner (i) ein Primerpaar, das mit einer Nukleinsäuresequenz hybridisieren kann, die die Nukleotidmutationssequenzen von Interesse umfasst, die jeweils für die Aminosäuremutationen HV69/70 del und Y144 del kodieren, (ii) ein Primerpaar, das mit einer Nukleinsäuresequenz hybridisieren kann, die die Nukleotidmutationssequenzen von Interesse umfasst, die jeweils für die Aminosäuremutationen E484K und N501Y kodieren, und (iii) ein Primerpaar, das mit einer Nukleinsäuresequenz hybridisieren kann, die die Nukleotidmutationssequenz von Interesse umfasst, die für die Aminosäuremutation P681H kodiert, umfasst,
wobei die Zusammensetzung ferner ein Oligonukleotid, das mit einer unter SARS-CoV-2-Wildtyp und -Varianten konservierten Nukleinsäuresequenz hybridisieren kann, und ein Oligonukleotid, das mit einer Nukleinsäuresequenz einer internen Kontrolle hybridisieren kann, umfasst.

9. Zusammensetzung nach Anspruch 8, wobei jedes PTO, das mit der Nukleotidmutationssequenz von Interesse hybridisieren kann, die für die Aminosäuremutationen E484K, N501Y und P681H kodiert, eine Nukleotidmutationsunterscheidungsstelle umfasst, die sich vom 5'-Ende seines 3'-Targeting-Abschnitts um 5 Nukleotide oder weniger entfernt befindet.

10. Zusammensetzung nach Anspruch 8, wobei sich die unter SARS-CoV-2-Wildtyp und -Varianten konservierte Nukleinsäuresequenz in einer Nukleinsäuresequenz befindet, die aus der Gruppe bestehend aus E-Gen, N-Gen, RdRP-Gen und S-Gen von SARS-COV-2 ausgewählt ist.

11. Zusammensetzung nach Anspruch 8, wobei die interne Kontrolle RNase P ist.

## Revendications

1. Procédé de détection de mutations de SARS-CoV-2 dans un échantillon comprenant une molécule d'acide nucléique, le procédé comprenant les étapes consistant à :
(a) incuber l'échantillon avec une composition pour détecter des mutations de SARS-CoV-2 ; et
(b) détecter les mutations de SARS-CoV-2,
dans lequel la composition comprend une pluralité de PTO (Probing and Tagging Oligonucleotide; oligonucléotide de sondage et de marquage) s'hybridant respectivement avec des séquences de mutation nucléotidique d'intérêt situées dans le gène S de SARS-CoV-2,
dans lequel le PTO comprend (i) une partie de ciblage en 3' comprenant une séquence nucléotidique d'hybridation complémentaire de la séquence d'acide nucléique cible, et (ii) une partie de marquage en 5' comprenant une séquence nucléotidique non complémentaire de la séquence d'acide nucléique cible ;
dans lequel les séquences de mutation nucléotidique d'intérêt codent chacune pour l'une quelconque des mutations d'acides aminés suivantes :
(i) HV69/70 del,
(ii) Y144 del,
(iii) E484K,
(iv) N501Y, et
(v) P681H,
dans lequel (1) ledit PTO capable de s'hybrider avec la séquence de mutation nucléotidique d'intérêt codant pour la mutation d'acides aminés HV 69/70 del comprend un site de discrimination de mutation nucléotidique au niveau de la région médiane de sa partie de ciblage en 3', et
(2) ledit PTO capable de s'hybrider avec la séquence de mutation nucléotidique d'intérêt codant pour la mutation d'acides aminés Y144 del comprend un site de discrimination de mutation nucléotidique au niveau de la région médiane de sa partie de ciblage en 3' ;
dans lequel (1) ledit PTO capable de s'hybrider avec la séquence de mutation nucléotidique d'intérêt codant pour la mutation d'acides aminés E484K comprend un site de discrimination de mutation nucléotidique dans une région d'extrémité 5' de sa partie de ciblage en 3',
(2) ledit PTO capable de s'hybrider avec la séquence de mutation nucléotidique d'intérêt codant pour la mutation d'acides aminés N501Y comprend un site de discrimination de mutation nucléotidique dans une région d'extrémité 5' de sa partie de ciblage en 3', et
(3) ledit PTO capable de s'hybrider avec la séquence de mutation nucléotidique d'intérêt codant pour la mutation d'acides aminés P681H comprend un site de discrimination de mutation nucléotidique dans une région d'extrémité 5' de sa partie de ciblage en 3',
dans lequel le site de discrimination de mutation nucléotidique comprend une séquence complémentaire de la variation nucléotidique sur l'acide nucléique cible,
dans lequel la composition comprend en outre (i) une paire d'amorces capables de s'hybrider avec une séquence d'acide nucléique comprenant les séquences de mutation nucléotidique d'intérêt codant respectivement pour les mutations d'acides aminés HV69/70 del et Y144 del, (ii) une paire d'amorces capables de s'hybrider avec une séquence d'acide nucléique comprenant les séquences de mutation nucléotidique d'intérêt codant respectivement pour les mutations d'acides aminés E484K et N501Y, et (iii) une paire d'amorces capables de s'hybrider avec une séquence d'acide nucléique comprenant la séquence de mutation nucléotidique d'intérêt codant pour la mutation d'acides aminés P681H,
dans lequel la composition comprend en outre un oligonucléotide capable de s'hybrider avec une séquence d'acide nucléique conservée parmi SARS-CoV-2 de type sauvage et des variants et un oligonucléotide capable de s'hybrider avec une séquence d'acide nucléique d'un témoin interne.

2. Procédé selon la revendication 1, dans lequel l'échantillon est un écouvillon, de la salive, ou une combinaison de ceux-ci ; dans lequel de préférence l'écouvillon est un écouvillon nasopharyngé, un écouvillon nasal, un écouvillon oropharyngé, un écouvillon salivaire, ou une combinaison de ceux-ci.

3. Procédé selon la revendication 1, dans lequel l'échantillon est un échantillon d'extrait brut qui n'a pas été soumis à une quelconque purification d'une séquence d'acide nucléique.

4. Procédé selon la revendication 1, dans lequel l'incubation comprend une transcription inverse et une réaction d'amplification d'acide nucléique.

5. Procédé selon la revendication 1, chaque PTO capable de s'hybrider avec la séquence de mutation nucléotidique d'intérêt codant pour les mutations d'acides aminés E484K, N501Y et P681H, comprend un site de discrimination de mutation nucléotidique situé à l'écart de l'extrémité 5' de sa partie de ciblage en 3' de 5 nucléotides ou moins.

6. Procédé selon la revendication 1, dans lequel la séquence d'acide nucléique conservée parmi SARS-CoV-2 de type sauvage et des variants est située dans une séquence d'acide nucléique choisie dans le groupe constitué du gène E, du gène N, du gène RdRP et du gène S de SARS-COV-2.

7. Procédé selon la revendication 1, dans lequel le témoin interne est la RNase P.

8. Composition pour détecter des mutations de SARS-CoV-2, la composition comprenant une pluralité de PTO (Probing and Tagging Oligonucleotide; oligonucléotide de sondage et de marquage) s'hybridant respectivement avec des séquences de mutation nucléotidique d'intérêt situées dans le gène S de SARS-CoV-2,
dans lequel le PTO comprend (i) une partie de ciblage en 3' comprenant une séquence nucléotidique d'hybridation complémentaire de la séquence d'acide nucléique cible, et (ii) une partie de marquage en 5' comprenant une séquence nucléotidique non complémentaire de la séquence d'acide nucléique cible ;
dans lequel les séquences de mutation nucléotidique d'intérêt codent chacune pour l'une quelconque des mutations d'acides aminés suivantes :
(i) HV69/70 del,
(ii) Y144 del,
(iii) E484K,
(iv) N501Y, et
(v) P681H,
dans lequel (1) ledit PTO capable de s'hybrider avec la séquence de mutation nucléotidique d'intérêt codant pour la mutation d'acides aminés HV 69/70 del comprend un site de discrimination de mutation nucléotidique au niveau de la région médiane de sa partie de ciblage en 3', et
(2) ledit PTO capable de s'hybrider avec la séquence de mutation nucléotidique d'intérêt codant pour la mutation d'acides aminés Y144 del comprend un site de discrimination de mutation nucléotidique au niveau de la région médiane de sa partie de ciblage en 3' ;
dans lequel (1) ledit PTO capable de s'hybrider avec la séquence de mutation nucléotidique d'intérêt codant pour la mutation d'acides aminés E484K comprend un site de discrimination de mutation nucléotidique dans une région d'extrémité 5' de sa partie de ciblage en 3',
(2) ledit PTO capable de s'hybrider avec la séquence de mutation nucléotidique d'intérêt codant pour la mutation d'acides aminés N501Y comprend un site de discrimination de mutation nucléotidique dans une région d'extrémité 5' de sa partie de ciblage en 3', et
(3) ledit PTO capable de s'hybrider avec la séquence de mutation nucléotidique d'intérêt codant pour la mutation d'acides aminés P681H comprend un site de discrimination de mutation nucléotidique dans une région d'extrémité 5' de sa partie de ciblage en 3',
dans lequel le site de discrimination de mutation nucléotidique comprend une séquence complémentaire de la variation nucléotidique sur l'acide nucléique cible,
dans lequel la composition comprend en outre (i) une paire d'amorces capables de s'hybrider avec une séquence d'acide nucléique comprenant les séquences de mutation nucléotidique d'intérêt codant respectivement pour les mutations d'acides aminés HV69/70 del et Y144 del, (ii) une paire d'amorces capables de s'hybrider avec une séquence d'acide nucléique comprenant les séquences de mutation nucléotidique d'intérêt codant respectivement pour les mutations d'acides aminés E484K et N501Y, et (iii) une paire d'amorces capables de s'hybrider avec une séquence d'acide nucléique comprenant la séquence de mutation nucléotidique d'intérêt codant pour la mutation d'acides aminés P681H,
dans lequel la composition comprend en outre un oligonucléotide capable de s'hybrider avec une séquence d'acide nucléique conservée parmi SARS-CoV-2 de type sauvage et des variants et un oligonucléotide capable de s'hybrider avec une séquence d'acide nucléique d'un témoin interne.

9. Composition selon la revendication 8, dans laquelle chaque PTO capable de s'hybrider avec la séquence de mutation nucléotidique d'intérêt codant pour les mutations d'acides aminés E484K, N501Y et P681H, comprend un site de discrimination de mutation nucléotidique situé à l'écart de l'extrémité 5' de sa partie de ciblage en 3' de 5 nucléotides ou moins.

10. Composition selon la revendication 8, dans laquelle la séquence d'acide nucléique conservée parmi SARS-CoV-2 de type sauvage et des variants est située dans une séquence d'acide nucléique choisie dans le groupe constitué du gène E, du gène N, du gène RdRP et du gène S de SARS-COV-2.

11. Composition selon la revendication 8, dans laquelle le témoin interne est la RNase P.
